# EUROPÄISCHE PATENTSCHRIFT

(11) **EP 3 515 898 B1**
(45) Veröffentlichungstag und Bekanntmachung des Hinweises auf die Patenterteilung: **11.11.2020**
(21) Anmeldenummer: 17768064.2
(22) Anmeldetag: 08.09.2017
(51) Int. Cl.: C07D 295/13, C07D 295/15, C08G 73/02

(54) **VERFAHREN ZUR HERSTELLUNG VON POLYAMINEN AUSGEHEND VON DINITRILEN UND/ODER AMINONITRILEN**
PROCESS FOR THE PREPARATION OF POLYAMINES STARTING FROM DINITRILES AND/OR AMINONITRILES
PROCEDE DE PREPARATION DE POLYAMINES A PARTIR DE DINITRILES ET/OU D'AMINONITRILES

(30) Priorität: 19.09.2016 EP 16189422
(43) Veröffentlichungstag der Anmeldung: 31.07.2019
(73) Patentinhaber: BASF SE, 67056 Ludwigshafen am Rhein (DE)
(72) Erfinder: REISSNER, Thomas, 67056 Ludwigshafen (DE); ALTENHOFF, Ansgar Gereon, 67056 Ludwigshafen (DE); MUELLER, Christian, 67056 Ludwigshafen (DE)
(74) Vertreter: BASF IP Association
(86) Internationale Anmeldenummer: PCT/EP2017/072602
(87) Internationale Veröffentlichungsnummer: WO 2018/050555

(56) Entgegenhaltungen:
- WO-A1-2014/131649
- WO-A1-2015/128021

## Beschreibung

Die vorliegende Erfindung betrifft ein Verfahren zur einstufigen Herstellung von Polyaminen durch Umsetzung von Verbindungen, die mindestens zwei Nitrilgruppen (Dinitril) oder mindestens eine Nitril- und eine Aminogruppe (Aminonitril) enthalten mit Wasserstoff in Gegenwart von heterogenen Übergangsmetall-Katalysatoren.

Polyamine stellen Oligomere oder Polymere dar, die wiederkehrende Ketten-Einheiten der Formeln R-NH-R' oder R-NR"-R' aufweisen. Moleküle, welche sich destillativ bis zu einem Druck von 1 mbar destillativ über Kopf entfernen lassen, werden in dieser Anmeldung als Oligomere bezeichnet. Diese Oligomere sind in die Synthese rückführbar und bestehen aus 2 bis 4 Wiederholungseinheiten des Monomers. Moleküle, die in dieser Weise destillativ nicht abtrennbar sind, werden als Polymere bezeichnet. Polyamine lassen sich beispielsweise ausgehend von zyklischen Iminen, wie Aziridinen, zyklischen Iminoethern, wie Oxazolinen und Aminoalkoholen herstellen wie es in WO 2014/131649 auf Seite 3 und 5 beschrieben ist.

Das vielseitigste Verfahren zur Herstellung von Polyaminen stellt die in WO2014/131649 beschriebene und der dort zitierten Literatur beschriebene Polykondensation von Diaminen in Gegenwart von Katalysatoren auf der Basis von Übergangsmetallen dar. Durch kontinuierliche Abtrennung des während der Polykondensation entstehenden Ammoniaks mit strömendem Wasserstoff lassen sich nach WO 2014/131649 Diamin-Umsatz, Polyamin-Ausbeute und mittleres Molgewicht deutlich steigern.

Die als Ausgangsverbindungen für die Herstellung von Polyaminen benötigten Diamine können durch katalytische reduktive Aminierung von Diolen, Dialdehyden oder Diketonen mit Ammoniak und Wasserstoff oder bevorzugt durch katalytische Hydrierung von Dinitrilen oder Aminonitrilen hergestellt werden (S. Gomez, J. A. Peters, Th. Maschmeyer, Adv. Synth. Catal. 2002, 344, Seiten 1037 bis 1057 und C. de Bellefon, P. Fouilloux, Catal. Rev. Sci. Eng., 36(3), Seiten 459 bis 506).

Für die aus dem Stand der Technik bekannten Verfahren zur Herstellung von Diaminen und/oder Aminonitrilen als Ausgangsverbindungen für die Herstellung von Polyaminen ist oft eine große Zahl von Reaktionsschritten notwendig. Dies kann zu beträchtlichen Investitions- und Herstellkosten führen.

So wird z.B. Adiponitril (ADN) zunächst in einem mehrstufigen Prozess ausgehend von Butadien und Blausäure hergestellt, dann in Gegenwart von Nickel-, Cobalt- oder Eisenkatalysatoren katalytisch zu Hexamethylendiamin (HMD) hydriert wie es von Hans-Jürgen Arpe in Industrielle Organische Chemie, 6. Auflage 2007, Wiley VCH-Verlag, Seite 275 beschrieben ist. Schließlich wird das HMD destillativ gereinigt. Erst dann kann das HMD durch Erhitzen in Gegenwart von Übergangsmetall-Katalysatoren in Polyamine überführt werden.

Die Polykondensation von Hexamethylendiamin zu Polyhexamethylenpolyamin gelingt in Gegenwart von Nickel- wie in WO 92/17437 oder Palladium-Katalysatoren wie in DE 2842264 beschrieben.
Nachteilig ist, dass für die Herstellung von Polyhexamethylenpolyaminen bei beiden Verfahren zwei Reaktionsschritte - eine Hydrierung und eine Polykondensation - mit unterschiedlichen Reaktionsbedingungen und Katalysatoren erforderlich sind. Hinzu kommt die destillative Aufarbeitung des HMD.

Weitere Nachteile bestehen darin, dass bei der Polykondensation des HMD in Gegenwart von Nickel und von Palladium als Katalysatoren farbige Polyhexamethylenpolyamine wie in WO 92/17437, Seite 7, Tabelle 1 und Beispiele 1 und 2 beschrieben, und in Gegenwart von Nickel Polyamine mit niedrigen mittleren Molekulargewichten wie in WO 92/17437, Seite 8 beschrieben, erhalten werden.

Auch die Herstellung von Polyaminen aus Aminonitrilen ist als zweistufiges Verfahren bekannt. In der deutschen Offenlegungsschrift DE 2605212 beschreiben die Beispiele 3 und 1 die Herstellung von Polypropylenpolyaminen aus Acrylnitril, Ammoniak und Wasserstoff.

Zunächst wird nach Beispiel 3 aus Acrylnitril und Ammoniak hergestelltes 3-Aminopropionitril bei 100°C und einem Wasserstoffdruck von 100 bar in Gegenwart von Ammoniak an einem fest angeordneten Cobaltkontakt zu Roh-1,3-Propandiamin hydriert.

Das in Beispiel 3 erhaltene Roh-1,3-Propandiamin wird dann gemäß Beispiel 1 bei 160°C und einem Wasserstoffdruck von 50 bar fortlaufend über einen Kontakt aus reduziertem Cobaltoxid geleitet. Man erhält 10 Gew.-% 1,3-Propandiamin, 12% Dipropylentriamin, 15% Tripropylentetramin und 70% Polypropylenpolyamin nicht beschriebener Zusammensetzung, bezogen auf eingesetztes Acrylnitril.

Nachteilig an dieser Verfahrensweise ist, dass die Herstellung von Polypropylenpolyamin aus 3-Aminopropionitril zweistufig bei unterschiedlichen Temperaturen und Drücken durchgeführt wird.

Aus DE 3 248 326 A1, Seite 5, Zeilen 12 bis 23 ist weiterhin bekannt, die Hydrierung von Mono-, Bis- oder Tris(2-cyanoethyl)aminen I, II und III oder deren Gemische unter Zusatz von 5 bis 400 Gew.-% eines Diamins durchzuführen. Die Hydrierung wird bei 60 bis 140°C, bevorzugt 70 bis 95°C und einem Druck von 60 bis 200 bar, bevorzugt 150 bis 200 bar in Gegenwart eines Cobalt-Katalysators durchgeführt, wie auf Seite 5, Zeilen 34 bis Seite 6, Zeile 2 beschrieben. Die Maximaltemperatur in Beispiel 1 beträgt 98°C, in Beispiel 3 96°C.

Bei der Hydrierung, wie in DE 3248326 beschrieben, entstehen die in Schema 1 gezeigten Amine IV, V oder VI, aber keine Polyamine. Der Diamin-Zusatz ist vorteilhaft, da gegebenenfalls während der Hydrierung freiwerdendes Acrylnitril durch das Diamin abgefangen wird. Dadurch wird verhindert, dass der Hydrierkatalysator durch freies Acrylnitril desaktiviert und seine Standzeit verringert wird.

Es bestand die Aufgabe, ein einstufiges und dadurch wirtschaftlicheres Verfahren zur Herstellung von Polyaminen ausgehend von Dinitrilen und/oder Aminonitrilen bereitzustellen. Bei diesem Verfahren sollten die katalytische Hydrierung der Nitrilgruppe und die Polykondensation der durch Hydrierung erhaltenen Diamine in einem engen Temperaturbereich in Gegenwart der gleichen Katalysatoren einstufig durchführbar sein. Die Polyamine sollten mit hohen mittleren Molekulargewichten mit guter Ausbeute und Reinheit (wenig Nebenprodukte, gute Farbzahl der Polyamine) anfallen. Hohes mittleres Molekulargewicht bedeutet einen Polymerisationsgrad Pn von 4 oder mehr, bevorzugt 10 oder mehr, insbesondere bevorzugt 15 oder mehr und ganz besonders bevorzugt 20 oder mehr. Der bei der Polykondensation gebildete Ammoniak sollte aus dem Verfahren ausgeschleust werden können.

Unter einstufiger Herstellung ist dabei zu verstehen, dass die Hydrierung der Nitrile und die anschließende Polykondensation der Diamine in Gegenwart von Wasserstoff ohne Zwischenaufarbeitung der zunächst gebildeten Diamine erfolgen und dass für die Hydrierung und Polykondensation kein Wechsel zu einem anderen Katalysator notwendig ist. Die Hydrierung und die Polykondensation werden dabei in ein bis zwei, bevorzugt in einem Reaktor und einem Temperaturbereich für beide Reaktionsschritte von 100°C bis 200°C durchgeführt.

Diese Aufgabe wird gelöst durch ein Verfahren zur Herstellung von Polyaminen, dadurch gekennzeichnet, dass man Verbindungen der Formel (I)

NC-Zₘ-(X)ₙ-Y (I)

wobei
- Z: der Gruppe CH₂-CH₂-NR- entspricht,
- m: = 0 oder 1 ist,
wobei im Fall von m=0 NC- direkt an X gebunden
im Fall von m=1 und n=1 NR an X gebunden ist und R= H oder CH₂-CH₂-CN ist, oder
im Falle von m=1 und n=0 R von NR entweder ausgewählt ist aus der Gruppe von CH₃, Phenyl, CH₂-CH₂-CN und CH₂-CH₂-CH₂-NH₂ oder der Stickstoff des NR Teil eines Heterocyclus ausgewählt aus der Gruppe von Piperazinen, (Benz)imidazolen, Pyrazolen, Triazolen und Diazepanen ist, bei dem ein anderer Stickstoff des Heterocycluses an Y gebunden ist,
- X: ausgewählt ist aus der Gruppe von mit Alkylgruppen substituierten oder unsubstituierten Methylen-, Ethylen-, Propylen-, Butylen-, Pentylen-, Hexylen-, Heptylen-, Octylen-, Nonanylen-, Decanylen-, Undecanylen-, Dodecanylengruppe, von mit Alkylgruppen substituierten oder unsubstituierten 1,2-, 1,3- oder 1,4- Benzylgruppen, 1,4'-Bicarbonylgruppen, 9,10-Anthracenylgruppen, mit Alkylgruppen substituierten oder unsubstituierten 1,2-, 1,3- oder 1,4-Cycloalkylgruppen, substituierter oder unsubstituierter Phenyl-N-Gruppe, von Piperazinen, (Benz)imidazolen, Pyrazolen, Triazolen und Diazepanen, wobei jeweils über die zwei Stickstoffatome des Rings die Bindung an CN bzw. Z und Y bzw. H erfolgt,einer bis zwei Methylengruppen, die durch lineares oder verzweigtes C₁- bis C₁₂-Alkyl, C₆- bis C₁₀-Aryl, C₇-bis C₁₂-Aralkyl, C₃- bis C₈-Cycloalkyl substituiert sein können und mindestens eine Methylengruppe durch O, NH oder NR ersetzt sein kann, wobei R die gleich Bedeutung wie oben hat und die C₁- bis C₁₂-Alkyl, C₆- bis C₁₀-Aryl, C₇- bis C₁₂-Aralkyl, C₃-bis C₈-Cycloalkylgruppen ihrerseits noch mit Alkylgruppen substituiert sein können,
- n: 0 oder eine natürliche Zahl von 1 bis 10 ist,
- Y,: für den Fall, dass m= 0 und n=1 oder höher ist, CN, CH₂-NH₂ oder CH₂-CH₂-CN ist oder
für den Fall, dass m=1 und n=1 oder höher ist, NH₂ oder NR-CH₂-CH₂-CN ist oder
für den Fall, dass m=1 und n=0 ist, H , CH₂-CH₂-CN, CH₂-CH₂-CH₂-NH₂ , mit Alkylgruppen substituierte oder unsubstituierte Benzyl oder Cycloalkylgruppe, ist oder
für den Fall, dass m=0 und n=0 ist, CH₂-CN, CH₂-CH₂-CN, CH₂-CH₂-CH₂-CN, CH₂-CH₂-CH₂-CH₂-CN, CH₂-CH₂-CH₂-CH₂-CH₂-CN, CH₂-NH₂, CH₂-CH₂-NH₂ CH₂-CH₂-CH2-NH₂ ist,
einstufig bei Temperaturen im Bereich von 100 bis 200°C und Drücken im Bereich von 20 bis 200 bar in Gegenwart von Wasserstoff und einem, falls mehrere Reaktoren verwendet werden, dem gleichen, heterogenen Katalysator, der ein oder mehrere Elemente der 8. Nebengruppe des Periodensystems enthält, umsetzt.

Bevorzugt ist das erfindungsgemäße Verfahren, wenn das gesamte Verfahren in einem einzigen Reaktor durchgeführt wird.

Bevorzugt ist das erfindungsgemäße, wenn jede Reaktionsstufe des Verfahrens beim gleichen Druck und der gleichen Temperatur durchgeführt wird.

Bevorzugt ist das erfindungsgemäße Verfahren, wenn die Verbindungen der Formel (I) Dinitrile der Formel

NC-(-X-)ₙ-CN (Ia)

oder

NC-CH₂-CH₂-NR-(-X-)ₙ-NH-CH₂-CH₂-CN (IIa)

sind, wobei R, n, X, die vorher genannte Bedeutung haben.

Bevorzugt ist das erfindungsgemäße Verfahren, wenn die Verbindung der Formel (I) Aminonitrile der Formel

NC-(-X)ₙ-CH₂NH₂, (Ib)

NC-(-X)ₙ (Ib')

oder

NC-CH₂-CH₂-NR-(-X)ₙ-NH₃ (IIb)

sind, wobei R, n und X, die vorher genannte Bedeutung haben.

Bevorzugt ist das erfindungsgemäße Verfahren, wenn die Elemente des eingesetzten heterogenen Katalysators ausgewählt sind aus der Gruppe von Co, Ni und/oder Cu.

Bevorzugt ist das erfindungsgemäße Verfahren, wenn der eingesetzte reduzierte heterogene Katalysator ein Co-Vollkontaktkatalysator mit bis zu 99 Gew.-% Co ist.

Bevorzugt ist das erfindungsgemäße Verfahren, wenn die eingesetzten Dinitrile ausgewählt sind aus der Gruppe von Malononitril, Succinonitril, Glutaronitril, Adiponitril, Suberonitril, 2-Methylmalononitril, 2-Methylsuccinonitril, 2-Methylglutaronitril, 1,2-, 1,3- und 1,4-Dicyanobenzol, 9,10-Anthracenodicarbonitril und 4,4'-Biphenyldicarbonitril.

Bevorzugt ist das erfindungsgemäße Verfahren, wenn die eingesetzten Aminonitrile ausgewählt sind aus der Gruppe von 6-Aminocapronitril, 4'-Aminobutyronitril, 3'-Aminopropionitril und 2'-Aminoacetonitril.

Bevorzugt ist das erfindungsgemäße Verfahren, wenn man einen Überschuss an Wasserstoff, bezogen auf die theoretisch zur Hydrierung der Nitrilgruppen notwendige Wasserstoff-Menge, von 1 bis 5 Molen in die Flüssig- oder Gasphase des ersten Reaktors (R1) einleitet und überschüssigen Wasserstoff zusammen mit Ammoniak, der bei der Umsetzung entsteht, aus dem letzten Reaktor ableitet.

Bevorzugt ist das erfindungsgemäße Verfahren, wobei der Ammoniak aus dem ausgeleiteten Ammoniak/Wasserstoffgemisch abgetrennt, aus dem Verfahren ausgeschleust und der abgetrennte Wasserstoff ganz oder teilweise in den Reaktor zurückgeführt wird.

Bevorzugt ist das erfindungsgemäße Verfahren, wenn die Trennung des Ammoniak/Wasserstoff-Gasgemisches durch Destillation oder nach Kühlung durch Phasentrennung erfolgt.

Bevorzugt ist das erfindungsgemäße Verfahren, wenn ein Teil des abgetrennten Ammoniaks in einen der Reaktoren, bevorzugt in den ersten Reaktor (R1), zurückgeführt wird.

Bevorzugt ist das erfindungsgemäße Verfahren, wenn das Verfahren diskontinuierlich oder kontinuierlich durchgeführt wird.

Bevorzugt ist das erfindungsgemäße Verfahren, wenn es sich bei dem oder den Reaktoren um Rohrreaktoren oder Rohrbündelreaktoren handelt.

Bevorzugt ist das erfindungsgemäße Verfahren, wenn das aus dem Polyamin-Rohaustrag nicht umgesetzte Dinitril/Aminonitril der Formel (I) und deren entsprechenden Diamine und Polyamin-Oligomere destillativ abgetrennt und in einen, bevorzugt den ersten Reaktor (R1) zurückgeführt werden.

In den Formeln (I), (Ia), (Ib), (Ib'), (IIa) und (IIb) bedeutet der "Spacer" X eine Gruppe ausgewählt aus der Gruppe von mit Alkylgruppen, substituierten oder unsubstituierten Methylen-, Ethylen-, Propylen-, Butylen-, Pentylen-, Hexylen-, Heptylen-, Octylen-, Nonanylen-, Decanylen-, Undecanylen-, Dodecanylengruppe, von mit Alkylgruppen substituierten oder unsubstituierten 1,2-, 1,3- oder 1,4- Benzylgruppen, 1,4'-Bicarbonylgruppen, 9,10-Anthracenylgruppen, mit Alkylgruppen substituierten oder unsubstituierten 1,2-, 1,3- oder 1,4-Cycloalkylgruppen, substituierte oder unsubstituierte Phenyl-N-Gruppe, von Piperazinen, (Benz)imidazolen, Pyrazolen, Triazolen und Diazepanen, wobei jeweils über die zwei Stickstoffatome des Rings die Bindung an CN bzw. Z und Y bzw. H erfolgt, einer bis zwei Methylengruppen, die durch lineares oder verzweigtes C₁- bis C₁₂-Alkyl, C₆- bis C₁₀-Aryl, C₇- bis C₁₂-Aralkyl, C₃- bis C₈-Cycloalkyl substituiert sein können und mindestens eine Methylengruppe durch O, NH oder NR ersetzt sein kann, wobei R die gleich Bedeutung wie oben hat und die C₁- bis C₁₂-Alkyl, C₆- bis C₁₀-Aryl, C₇- bis C₁₂-Aralkyl, C₃- bis C₈-Cycloalkylgruppen ihrerseits noch mit Alkylgruppen substituiert sein können.
Z steht für CH₂-CH₂-NR.
Der Index m kann 0 oder 1 sein, wobei im Fall von m=0 NC- direkt an X gebunden ist, im Fall von m=1 und n=1 NR an X gebunden ist und R dann H oder CH₂-CH₂-CN ist oder im Fall von m=1 und n=0 R von NR entweder ausgewählt ist aus der Gruppe von CH₃, Phenyl, CH₂-CH₂-CN und CH₂-CH₂-CH₂-NH₂ oder der Stickstoff von NR Teil eines Heterocyclus ist, bei dem R mindestens ein weiteres Stickstoffatom enthält und dieses weitere Stickstoffatom an Y gebunden ist.
Y ist von m und n abhängig. Wenn m=0 ist, muss n=1 oder höher sein und dann ist Y ausgewählt aus der Gruppe von CN, CH₂-NH₂ und CH₂-CH₂-NH₂. Wenn m=1 und n=1 oder höher ist, ist Y ausgewählt aus der Gruppe von NH₂ und NR-CH₂-CH₂-CN, wobei R die obige Bedeutung hat. Wenn m=1 und n=0 ist, ist Y ausgewählt aus der Gruppe von H, CH₂-CH₂-CN und CH₂-CH₂-CH₂-NH₂, mit Alkylgruppen substituierte oder unsubtituierte Benzyl oder Cycloalkygruppe ist oder für den Fall, dass m=0 und n=0 ist, CH₂-CN, CH₂-CH₂-CN, CH₂-CH₂-CH₂-CN, CH₂-CH₂-CH₂-CH₂-CN, CH₂-CH₂-CH₂-CH₂-CH₂-CN, CH₂-NH₂, CH₂-CH₂-NH₂, CH₂-CH₂-CH₂-NH₂ ist.
Der Index n ist entweder 0 oder eine natürliche Zahl von 1 bis 10.

Die Verbindungen der Formel (I) enthalten Dinitrile der Formel (Ia) und (IIa) sowie Aminonitrile der Formel (Ib), (Ib') und (IIb).

### Dinitrile der Formel (Ia)

Als Ausgangsverbindungen für das erfindungsgemäße Verfahren benötigte Dinitrile (la) lassen sich durch Umsetzung von entsprechenden alpha,omega- oder alpha,beta-Dihalogenverbindungen mit Alkalicyaniden herstellen, wobei allerdings äquimolare Mengen an Alkalihalogeniden als Nebenprodukte entstehen. Sie sind weiterhin zugänglich durch Dehydratisierung von Carbonsäureamiden und Aldoximen.

Das Faservorprodukt Adiponitril wird großtechnisch nach drei unterschiedlichen Verfahren hergestellt: durch i) Umsetzung von Adipinsäure mit Ammoniak, ii) elektrochemische Dimerisierung von Acrylnitril oder iii) durch Hydrocyanierung von Butadien bzw. 3-Pentennitril mit Blausäure (Ullmann's Encyclopedia of Industrial Chemistry, 6. Auflage, Band 16, Seite 434).

Bevorzugte Dinitrile (la) sind Verbindungen, in denen für X 1,2-, 1,3- oder 1,4-Benzylgruppen alkylsubstituiert oder unsubstituiert, 1,2-, 1,3-, oder 1,4-Cycloalyklgruppen alkylsubstituiert oder unsubstituiert, Methylengruppen, die durch C₁- bis C₅-Alkylreste substituiert sein können und bei denen n 1 bis 10 bedeutet. Bevorzugte Dinitrile der Formel (la) sind ausgesucht aus der Gruppe von Malononitril, Succinonitril, Glutaronitril, Adiponitril, Suberonitril, 2-Methylmalononitril, 2-Methylsuccinonitril, 2-Methylglutaronitril, 1,2-, 1,3- und 1,4-Dicyanobenzol, 9,10-Anthracenodicarbonitril und 4,4'-Biphenyldicarbonitril. Besonders bevorzugt sind Adiponitril, Succinonitril, Malononitril und 2-Methylglutaronitril. Ganz besonders bevorzugte Dinitrile der Formel (la) sind Adiponitril und Malononitril.

### Aminonitrile der Formel (Ib) und (Ib')

Als Ausgangsverbindungen für das erfindungsgemäße Verfahren benötigte Aminonitrile (Ib) sind beispielsweise durch partielle Hydrierung von Dinitrilen, z.B. durch partielle Hydrierung von Adiponitril zu 6-Aminocapronitril in Gegenwart von Eisen, Cobalt oder Nickel-Katalysatoren zugänglich wie es in US 6 297 394 beschrieben ist. Aminoacetonitrile lassen sich durch Umsetzung von Formaldehyd mit Cyanwasserstoff und Ammoniak oder Alkylaminen herstellen, wie es in US 5 008 428 beschrieben ist. Aminopropionitrile sind durch Umsetzung von Acrylnitril oder Methacrylnitril mit Ammoniak oder Aminen zugänglich wie es in DE 2 605 212 oder DE 3 248 326 beschrieben ist. Bevorzugt sind solche Aminonitrile die aus der Reaktion eines primären Amins oder eines sekundäres Amin, das noch wenigstens eine weitere primäre Aminfunktion enthält, mit 1 Mol Acrylnitril entstehen. Besonders bevorzugt sind 3-Aminopropionitril, N-Methylpropionitril, H₂N(CH₂)₂-NH-(CH₂)₂-CN, 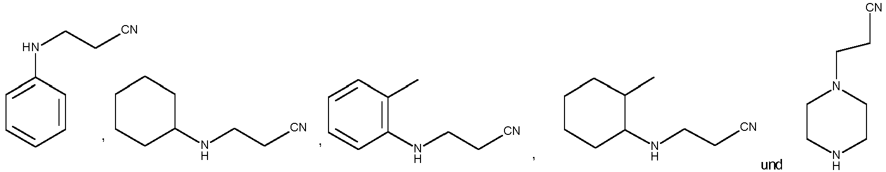 Ganz besonders bevorzugte Dinitrile der Formel (Ib) und (Ib') sind 3-Aminopropionitril, N-Methylpropionitril und 3-Piperazin-1-ylpropanenitril.

### Dinitrile (IIa) und Aminonitrile (IIb)

Dinitrile (IIa) und Aminonitrile (IIb) werden durch Umsetzung von Diaminen mit einem Mol oder zwei Molen Acrylnitril oder Methacrylnitril erhalten. Diese als Cyanethylierung bekannte Umsetzung kann z.B. in wässriger Lösung durchgeführt werden und ist in March's Advanced Organic Chemistry, 6. Auflage 2007, Seite 1008, Wiley-Interscience beschrieben.

Als "Spacer-Diamine" X in den Formeln (IIa) und (IIb) sind Teilstücke von aliphatischen Diaminen, oligomere Polyamine, die aus 2 bis 5 Amineinheiten bestehen, alicyclische und cyclische aromatische Diamine geeignet, deren zwei Aminfunktionen durch die Kopplung an Z und Y aus der Formel (I) entfallen sind. X ist besonders bevorzugt ein Teilstück eines der folgenden Diamine ohne die zwei Aminfunktionen ausgewählt aus der Gruppe von Ethylendiamin, 1,3-Propylendiamin, 1,2-Propylendiamin, 1,4-Butylendiamin, 1,2-Butylendiamin, 1,5-Diaminopentan, 1,2-Diaminopentan, 1,5-Diamino-2-methylpentan, 1,6-Diaminohexan, 1,2-Diaminohexan 1,7-Diaminoheptan, 1,2-Diaminoheptan, 1,8-Diaminooctan, 1,2-Diaminooctan, 1,9-Nonamethylendiamin, 1,10-Decamethylendiamin, 1,2-Diaminodekan, 1,11-Undecamethylendiamin, 1,2-Diaminoundekan, 1,12-Dodecamethylendiamin, 1,2-Diaminododekan, 2,2-Dimethylpropan-1,3-diamin, 4,7,10-Trioxatridekan-1,13-diamin, 4,9-Dioxadodekan-1,12-diamin, N,N-Bis(3-aminopropyl)methylamin, N,N-Bis-(3-Aminopropyl)ethylendiamin, 3-(2-Aminoethylamino)propylamin, Diethylentriamin (DETA), Triethylentetramin (TETA), Tetraethylenpentamin (TEPA), Di-1,3-propylentriamin, Tri-1,3-propylentetramin und Tetra-1,3-propylenpentamin, Di-1,2-propylentriamin, Tri-1,2-propylentetramin und Tetra-1,2-propylenpentamin, Dihexamethylentriamin, Trihexamethylentetramin und Tetrahexamethylenpentamin, 3,3'-Dimethyl-4,4'-diaminodicyclohexylmethan, 4,4-Diaminodicyclohexylmethan, Isophorondiamin, 1,3-Bis-(aminomethyl)-cyclohexan, Bis-(4-amino-cyclohexyl)-methan, Bis-(4-amino-3,5-dimethyl-cyclohexyl)-methan oder Bis-(4-amino-3-methyl-cyclohexyl)-methan, 3-(Cyclohexylamino)propylamin, Piperazin, meta-Xylendiamin (MXDA), 4-Methylcyclohexan-1,3-diamin, 3-Methylcyclohexan-1,2-diamin, 2-Methylcyclohexan-1,3-diamin, 4-Methylcyclohexan-1,2-diamin, 2-Methylcyclohexan-1,4-diamin, 5-Methylcyclohexan-1,3-diamin und ihre cis und trans-Isomere oder Isomere von Aminobenzylamin (2-Aminobenzylamin, 4-Aminobenzylamin), 4-(2-Aminoethyl)anilin, m-Xylylenediamin, o-Xylylenediamin, oder 2,2-Biphenyldiamine, oder Oxydianiline, wie z.B. 4,4-Oxydianilin, Isomere von Diaminofluoren, Isomere von Diaminophenanthren und 4,4-Ethylendianilin, Jeffamine der Fa. Huntsman, insbesondere Jeffamin D230, Jeffamin D400, Jeffamin D2000, Jeffamin D4000, Jeffamin ED600, Jeffamin ED900, Jeffamin ED2003, Jeffamin EDR148 und Jeffamin EDR176.
X ist ganz besonders bevorzugt ein Teilstück eines der folgenden Diamine ohne die zwei Aminfunktionen ausgewählt aus der Gruppe von 1,3-Propylendiamin, 1,6-Diaminohexan, 1,8-Diaminooctan, 1,10-Decamethylendiamin, 1,12-Dodecamethylendiamin, 2,2-Dimethylpropan-1,3-diamin, N,N-Bis(3-aminopropyl)methylamin, N,N-Bis-(3-Aminopropyl)ethylendiamin, Di-1,3-propylentriamin, Tri-1,3-propylentetramin, Dihexamethylentriamin, 4,4-Diaminodicyclohexylmethan, Isophorondiamin, 1,3-Bis-(aminomethyl)-cyclohexan, Piperazin, meta-Xylendiamin (MXDA), Bis-(4-amino-cyclohexyl)-methan, 3-(Cyclohexylamino)propylamin, 4-Methylcyclohexan-1,3-diamin, 3-Methylcyclohexan-1,2-diamin, 2-Methylcyclohexan-1,3-diamin, 4-Methylcyclohexan-1,2-diamin, 2-Methylcyclohexan-1,4-diamin, 5-Methylcyclohexan-1,3-diamin und ihre cis und trans-Isomere, m-Xylylenediamin, o-Xylylenediamin, Jeffamine der Fa. Huntsman, insbesondere Jeffamin D230, Jeffamin D400, Jeffamin D2000, Jeffamin D4000, Jeffamin ED600, Jeffamin ED900, Jeffamin ED2003, Jeffamin EDR148 und Jeffamin EDR176. X ist insbesondere ganz besonders bevorzugt ein Teilstück eines der folgenden Diamine ohne die zwei Aminfunktionen ausgewählt aus der Gruppe von 1,3-Propylendiamin, 1,6-Diaminohexan, N,N-Bis(3-aminopropyl)methylamin, N,N-Bis-(3-Aminopropyl)ethylendiamin, Isophorondiamin, Piperazin, meta-Xylendiamin (MXDA), 4-Methylcyclohexan-1,3-diamin, 3-Methylcyclohexan-1,2-diamin, 2-Methylcyclohexan-1,3-diamin, 4-Methylcyclohexan-1,2-diamin, 2-Methylcyclohexan-1,4-diamin, 5-Methylcyclohexan-1,3-diamin und ihre cis und trans-Isomere.

### Katalysatoren

Als Katalysatoren für die Umsetzung von Dinitrilen (la) und (IIa) und Aminonitrilen (Ib),(Ib') und (IIb) zu Polyaminen können insbesondere Katalysatoren eingesetzt werden, die ein oder mehrere Elemente der 8. Nebengruppe des Periodensystems (Fe, Co, Ni, Ru, Rh, Pd, Os, Ir, Pt), bevorzugt Co, Ni, Ru, Cu und/oder Pd, besonders bevorzugt Co, Ni und/oder Cu, ganz besonders bevorzugt Cobalt enthalten. Diese Elemente werden nachfolgend auch als katalytisch aktive Metalle bezeichnet.

Die oben genannten Katalysatoren können mit Promotoren, beispielsweise mit Chrom, Eisen, Kobalt, Mangan, Molybdän, Titan, Zinn, Metallen der Alkaligruppe, Metallen der Erdalkaligruppe und/oder Phosphor dotiert sein.

Als Katalysatoren können bevorzugt sogenannte Skelett-Katalysatoren (auch als Raney-Typ bezeichnet, nachfolgend auch: Raney-Katalysator) eingesetzt werden, die durch Auslaugen (Aktivierung) einer Legierung aus Katalysator, reaktivem Metall und einer weiteren Komponente (bevorzugt Al) mit Alkali erhalten werden. Bevorzugt werden Raney-Nickel- oder Raney-Cobalt-Katalysatoren eingesetzt.
Als Katalysatoren werden weiterhin bevorzugt Trägerkatalysatoren eingesetzt. Bevorzugte Trägermaterialien sind Aktivkohle, Al₂O₃, TiO₂, ZrO₂ und SiO₂.

Im Falle von überwiegend Cobalt enthaltenden Katalysatoren sind ganz besonders Cobaltvollkatalysatoren geeignet, die bis zu 99% Cobalt, aber keinen Träger enthalten und in EP-A1 636 409 beschrieben sind.

Ganz besonders bevorzugt werden in das erfindungsgemäße Verfahren Katalysatoren eingesetzt, die durch Reduktion von sogenannten Katalysator-Vorläufern hergestellt werden.

Der Katalysator-Vorläufer enthält eine aktive Masse, die eine oder mehrere katalytisch aktive Komponenten, ggf. Promotoren und optional ein Trägermaterial enthält.
Bei den katalytisch aktiven Komponenten handelt es sich um sauerstoffhaltige Verbindungen der oben genannten katalytisch aktiven Metalle, beispielsweise um deren Metalloxide bzw. Hydroxide, wie CoO, NiO, CuO und/oder deren Mischoxide.

In der Regel tritt erst nach erfolgter Reduktion eine katalytische Aktivität in der erfindungsgemäßen Umsetzung auf.

Besonders bevorzugt sind Katalysator-Vorläufer, die ein oder mehrere Oxide der Elemente Cu, Co und Ni enthalten. Beispielsweise die in EP-A-0636409 offenbarten Oxidgemische, die vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,2 bis 15 Gew.-% Alkali, berechnet als M₂O (M = Alkali), enthalten, oder
in EP-A-0742045 offenbarte Oxidgemische, die vor der Reduktion mit Wasserstoff 55 bis 98 Gew.-% Co, berechnet als CoO, 0,2 bis 15 Gew.-% Phosphor, berechnet als H₃PO₄, 0,2 bis 15 Gew.-% Mangan, berechnet als MnO₂, und 0,05 bis 5 Gew.-% Alkali, berechnet als M₂O (M = Alkali) enthalten, oder
in EP-A-696572 offenbarte Oxidgemische, die vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthalten, beispielsweise der in Loc. cit., Seite 8, offenbarte Katalysator mit der Zusammensetzung 31,5 Gew.-% ZrO₂, 50 Gew.-% NiO, 17 Gew.-% CuO und 1,5 Gew.-% MoO₃, oder
in EP-A-963975 offenbarte Oxidgemische, die vor der Reduktion mit Wasserstoff 22 bis 45 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis grösser 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂O₃ bzw. MnO₂, und keine sauerstoffhaltigen Verbindungen des Molybdäns enthält, beispielsweise der in Loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO, enthalten.

In einer ganz besonders bevorzugten Ausführungsform sind 50 bis 100 mol%, besonders bevorzugt 60 bis 99 mol% und ganz besonders bevorzugt 75 bis 98 mol% der in der katalytisch aktiven Masse enthaltenen katalytisch aktiven Metalle ein oder mehrere Metalle ausgewählt aus der Gruppe bestehend aus Cu, Co und Ni.

Das molare Verhältnis der Atome der Komponenten der aktiven Masse zueinander kann mittels bekannten Methoden der Elementaranalyse, beispielsweise der Atomabsorptionsspektrometrie (AAS), der Atomemissionsspektrometrie (AES), der Röntgenfluoreszenzanalyse (RFA) oder der ICP-OES (Inductively Coupled Plasma Optical Emission Spectrometry) gemessen werden. Das molare Verhältnis der Atome der Komponenten der aktiven Masse zueinander kann aber auch rechnerisch bestimmt werden, beispielsweise dadurch, dass die Einwaagen der verwendeten Verbindungen, die die Komponenten der aktiven Masse enthalten, bestimmt werden und die Anteile der Atome der Komponenten der aktiven Masse aufgrund der bekannten Stöchiometrie der eingesetzten Verbindungen bestimmt werden, so dass das atomare Verhältnis aus den Einwaagen und der stöchiometrischen Formel der eingesetzten Verbindung berechnet werden kann. Natürlich kann die stöchiometrische Formel der eingesetzten Verbindungen auch experimentell bestimmt werden, beispielsweise durch ein oder mehrere der oben genannten Methoden.

Je nach durchgeführtem Verfahren (Suspensionspolymerisation, Wirbelschichtverfahren, Festbettpolymerisation) werden die Katalysatoren als Pulver, Splitt oder Formkörper (bevorzugt Extrudate oder Tabletten) eingesetzt.

Die Katalysatoren bzw. Katalysatorvorläufer werden bevorzugt in Form von Formkörpern in das erfindungsgemäße Verfahren eingesetzt.

Als Formkörper eignen sich solche mit beliebiger Geometrie bzw. Form. Bevorzugte Formen sind Tabletten, Ringe, Zylinder, Sternstränge, Wagenräder oder Kugeln. Besonders bevorzugt sind Tabletten, Ringe, Zylinder, Kugeln oder Sternstränge. Ganz besonders geeignet sind Tabletten, Ringe, Sternstränge und Zylinder

### Tränkung

In einer bevorzugten Ausführungsform werden die Katalysatoren in Form von Formkörpern in das erfindungsgemäße Verfahren eingesetzt, die durch Tränkung (Imprägnierung) von Trägermaterialien hergestellt werden, die oben genannte Geometrie aufweisen oder die nach der Tränkung zur Formkörpern, die die oben genannte Geometrie aufweisen verformt werden.

Als Trägermaterialien kommen beispielsweise Kohlenstoff, wie Graphit, Ruß, Graphen, Kohlenstoffnanoröhrchen und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die Tränkung der obengenannten Trägermaterialien kann nach den üblichen Verfahren, wie es in A. B. Stiles, Catalyst Manufacture - Laboratory and Commercial Preparations, Marcel Dekker, New York, 1983 beschrieben ist, erfolgen, beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.

Die Calcinierung wird im Allgemeinen bei Temperaturen zwischen 300 und 800°C, vorzugsweise 350 bis 600°C, insbesondere bei 450 bis 550°C ausgeführt.

Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.
Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

Bevorzugt werden Trägermaterialien eingesetzt, die bereits die zuvor beschriebene bevorzugte Geometrie der Formkörper aufweisen.

Es ist jedoch auch möglich Trägermaterialien einzusetzen, die als Pulver oder Splitt vorliegen, und getränkten Trägermaterialien einer Formgebung zu unterziehen.

So kann beispielsweise das imprägnierte und getrocknete bzw. calcinierte Trägermaterial konditioniert werden.

Die Konditionierung kann beispielsweise dadurch erfolgen, dass man das getränkte Trägermaterial durch Vermahlen auf eine bestimmte Korngröße einstellt.

Nach der Vermahlung kann das konditionierte, getränkte Trägermaterial mit Formhilfsmitteln wie Graphit, oder Stearinsäure vermischt werden und zu Formkörpern weiterverarbeitet werden.

Gängige Verfahren der Formgebung sind beispielsweise in Ullmann's Encyclopedia Electronic Release 2000, Kapitel: "Catalysis and Catalysts", Seiten 28-32 und von Ertl et al., Knözinger, Weitkamp, Handbook of Heterogeneous Catalysis, VCH Weinheim, 1997, Seiten 98 ff beschrieben.

Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

### Mischfällung

In einer weiteren bevorzugten Ausführungsform werden Formkörper in das erfindungsgemäße Verfahren eingesetzt, die durch eine gemeinsame Fällung (Mischfällung) aller ihrer Komponenten hergestellt werden und die so ausgefällten Katalysatorvorläufer einer Formgebung unterzogen werden.

Dazu wird in der Regel eine lösliche Verbindung der entsprechenden aktiven Komponente, der Dotierelemente und ggf. eine lösliche Verbindung eines Trägermaterials in einer Flüssigkeit in der Wärme und unter Rühren so lange mit einem Fällungsmittel versetzt, bis die Fällung vollständig ist.

Als Flüssigkeit wird in der Regel Wasser eingesetzt.

Als lösliche Verbindung der aktiven Komponenten kommen üblicherweise die entsprechenden Metallsalze, wie die Nitrate, Sulfate, Acetate oder Chloride, der voranstehend genannten Metalle in Betracht.

Als lösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als lösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

Üblicherweise werden bei den Fällungsreaktionen die löslichen Verbindungen durch Zugabe eines Fällungsmittels als schwer- oder unlösliche, basische Salze gefällt. Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.

Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100°C, besonders 30 bis 90°C, insbesondere bei 50 bis 70°C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 350 bis 600°C, insbesondere bei 450 bis 550°C ausgeführt.

Nach der Calcinierung werden die durch Fällungsreaktionen erhaltenen pulverförmigen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt.

Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise in Ullmann's Encyclopedia Electronic Release 2000, Kapitel: "Catalysis and Catalysts", Seiten 28-32 und von Ertl et al. Ertl, Knözinger, Weitkamp, Handbook of Heterogeneous Catalysis, VCH Weinheim, 1997, Seiten 98 ff beschrieben.

Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

### Auffällung

In einer weiteren, bevorzugten Ausführungsform können die Formkörper durch Auffällung hergestellt werden.

Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).

Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.
Das Trägermaterial liegt in der Regel als Pulver oder Splitt vor.
Als Flüssigkeit, in der das Trägermaterial suspendiert wird, wird üblicherweise Wasser eingesetzt.

Als lösliche Verbindungen kommen die voranstehend genannten löslichen Verbindungen der aktiven Komponenten bzw. der Dotierelemente in Betracht.

Die Fällungsreaktionen können z.B. bei Temperaturen von 20 bis 100°C, besonders 30 bis 90°C, insbesondere bei 50 bis 70°C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 350 bis 600°C, insbesondere bei 450 bis 550°C ausgeführt.

Nach der Calcinierung werden die durch Fällungsreaktionen erhaltenen pulverförmigen Katalysatorvorläufer üblicherweise konditioniert.

Die Konditionierung kann beispielsweise dadurch erfolgen, dass man den Fällungskatalysator durch Vermahlen auf eine bestimmte Korngröße einstellt.

Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.

Gängige Verfahren der Formgebung sind beispielsweise in Ullmann's Encyclopedia Electronic Release 2000, Kapitel: "Catalysis and Catalysts", Seiten 28-32 und von Ertl et al., Knözinger, Weitkamp, Handbook of Heterogeneous Catalysis, VCH Weinheim, 1997, Seiten 98 ff beschrieben.

Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.

Durch den Prozess der Formgebung können Formkörper mit der oben genannten Geometrie erhalten werden.

Nach der Konditionierung bzw. Formgebung erfolgt in der Regel eine Temperung. Die Temperaturen bei der Temperung entsprechen üblicherweise den Temperaturen bei der Calcinierung.

### Reduktion

Formkörper, die durch Tränkung oder Fällung (Auffällung bzw. Mischfällung) hergestellt wurden, enthalten in der Regel die katalytisch aktiven Komponenten nach erfolgter Calcinierung in Form ihrer sauerstoffhaltigen Verbindungen, beispielsweise deren Metalloxide bzw. Hydroxide, wie CoO, NiO, CuO und/oder deren Mischoxide (Katalysatorvorläufer).

Die Katalysatorvorläufer, die wie voranstehend beschrieben durch Imprägnierung oder Fällung (Auffällung oder Mischfällung) hergestellt wurden, werden im Allgemeinen nach der Calcinierung bzw. Konditionierung reduziert. Durch die Reduktion wird der Katalysatorvorläufer in der Regel in seine katalytisch aktive Form umgewandelt.

Die Reduktion des Katalysatorvorläufers kann bei erhöhter Temperatur in einem bewegten oder unbewegten Reduktionsofen durchgeführt werden.
Als Reduktionsmittel wird üblicherweise Wasserstoff oder ein Wasserstoff enthaltendes Gas eingesetzt.

Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Der Wasserstoffstrom kann auch als Kreisgas in die Reduktion zurückgeführt werden, ggf. vermischt mit Frisch-Wasserstoff und ggf. nach Entfernen von Wasser durch Kondensation.

Die Reduktion des Katalysatorvorläufers erfolgt bevorzugt in einem Reaktor, in dem die Formkörper als Festbett angeordnet sind. Besonders bevorzugt erfolgt die Reduktion des Katalysatorvorläufers in demselben Reaktor in dem die nachfolgende Umsetzung erfolgt.
Weiterhin kann die Reduktion des Katalysatorvorläufers in einem Wirbelschichtreaktor in der Wirbelschicht erfolgen.

Die Reduktion des Katalysatorvorläufers erfolgt in der Regel bei Reduktionstemperaturen von 50 bis 600°C, insbesondere von 100 bis 500°C, besonders bevorzugt von 150 bis 450°C. Der Wasserstoffpartialdruck beträgt in der Regel von 1 bis 300 bar, insbesondere von 1 bis 200 bar, besonders bevorzugt von 1 bis 100 bar, wobei sich die Druckangaben hier und im Folgenden auf den absolut gemessenen Druck beziehen.

Die Dauer der Reduktion beträgt bevorzugt 1 bis 20 Stunden, und besonders bevorzugt 5 bis 15 Stunden.

Während der Reduktion kann ein Lösungsmittel zugeführt werden, um entstehendes Reaktionswasser abzuführen und/oder um beispielsweise den Reaktor schneller aufheizen zu können und/oder während der Reduktion die Wärme besser abführen zu können. Das Lösungsmittel kann hierbei auch überkritisch zugeführt werden.

Als geeignete Lösungsmittel können die zuvor beschriebenen Lösungsmittel eingesetzt werden. Bevorzugte Lösungsmittel sind Wasser; Ether wie Methyltertbutylether, Ethyltertbutylether, Dioxan oder Tetrahydrofuran. Besonders bevorzugt sind Wasser oder Tetrahydrofuran. Als geeignete Lösungsmittel kommen ebenfalls geeignete Mischungen in Betracht.

Der so erhaltene Formkörper kann nach der Reduktion unter inerten Bedingungen gehandhabt werden. Bevorzugt kann der Formkörper unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Katalysator eingesetzt wird. Gegebenenfalls muss der Katalysator vor Beginn der eigentlichen Reaktion dann von der inerten Flüssigkeit befreit werden.

Die Lagerung des Katalysators unter inerten Substanzen ermöglicht eine unkomplizierte und ungefährliche Handhabung und Lagerung des Formkörpers.

Der Formkörper kann nach der Reduktion aber auch mit einem Sauerstoff enthaltenden Gasstrom wie Luft oder einem Gemisch von Luft mit Stickstoff in Kontakt gebracht werden.
Dadurch wird ein passivierter Formkörper erhalten. Der passivierte Formkörper weist im Allgemeinen eine schützende Oxidschicht auf. Durch diese schützende Oxidschicht wird die Handhabung und Lagerung des Katalysators vereinfacht, so dass beispielsweise der Einbau des passivierten Formkörpers in den Reaktor vereinfacht wird. Ein passivierter Formkörper wird bevorzugt vor dem Inkontaktbringen mit den Edukten wie oben beschrieben durch Behandlung des passivierten Katalysators mit Wasserstoff oder einem Wasserstoff enthaltenden Gas reduziert. Die Reduktionsbedingungen entsprechen im Allgemeinen den Reduktionsbedingungen, die bei der Reduktion der Katalysatorvorläufer angewandt werden. Durch die Aktivierung wird in der Regel die schützende Passivierungsschicht aufgehoben.

### Verfahrensparameter

Die erfindungsgemäße Umsetzung von Dinitrilen und Aminonitrilen der Formel (la), (Ib), (Ib'), (IIa) und (IIb) mit Wasserstoff zu Polyaminen erfolgt im Temperaturbereich von 100 bis 200°C, bevorzugt von 130 bis 190°C, besonders bevorzugt 150 bis 180°C.

Die Umsetzung wird bevorzugt bei einem Druck durchgeführt, bei dem das Edukt-/ Produktgemisch im flüssigen Zustand vorliegt. Sie erfolgt in Gegenwart von Wasserstoff bei einem Druck von 20 bis 200 bar, bevorzugt 50 bis 190 bar, besonders bevorzugt 50 bis 180 bar.

Die Katalysator-Belastung bei kontinuierlicher Fahrweise liegt im Allgemeinen bei 0,1 bis 2, bevorzugt 0,1 bis 1,5, besonders bevorzugt 0,1 bis 1,2 kg Edukt pro I Katalysator und Stunde jeweils bei vorherrschender Reationstemperatur und -druck.

### Zufuhr der Dinitrile, Trinitrile und/oder Aminonitrile der Formel (Ia), (IIa), (Ib), (Ib'), (IIb)

Je nach gewünschter Zusammensetzung des Polyamins können die Ausgangsverbindungen (la), (IIa), (Ib), (Ib'), (IIb) einzeln oder als Gemische aus zwei, drei oder vier der Ausgangsverbindungen dem Reaktor (R1) zugeführt werden.

Bei diskontinuierlicher Fahrweise werden Dinitrile, Trinitrile und/oder Aminonitrile der Formel (Ia), (IIa), (Ib), (Ib'), (IIb) bevorzugt im Reaktor vorgelegt. Sie können mit geeigneten Fördervorrichtungen, z.B. Flüssigkeitspumpen, Vakuumförderern oder pneumatischen Förderern in den Reaktor gefördert werden. Geeignete Vorrichtungen zum Befüllen eines Reaktors, in Abhängigkeit vom Aggregatzustand der zu fördernden Substanz, sind dem Fachmann bekannt.

Die Ausgangsverbindungen werden bevorzugt in flüssigem Zustand in den Reaktor (R1) gefördert. Hierzu kann es notwendig sein, sie auf eine Temperatur oberhalb ihres Schmelz- bzw. Erstarrungspunkts zu erwärmen und/oder unter einem Druck zu arbeiten, bei dem sie in flüssigem Zustand vorliegen. Weiterhin kann es bevorzugt sein, die Ausgangsverbindungen in einem Lösungsmittel zu lösen.

Geeignete Lösungsmittel sind beispielsweise Flüssigkeiten, die sich unter den Bedingungen der Umsetzung weitestgehend inert verhalten.

Bevorzugte Flüssigkeiten sind C4- bis C12-Dialkylether, wie Diethylether, Diisopropylether, Dibutylether, tert-Butylmethylether oder cyclische C4- bis C12-Ether, wie Tetrahydrofuran, 2- und 3-Methyltetrahydrofuran oder Dioxan, Dimethoxyethan, Diethylenglykoldimethylether oder Kohlenwasserstoffe wie Pentan, Hexan, Heptan, 2,2,4-Trimethylpentan, Octan, Cyclohexan, Methylcyclohexan, Xylol, Toluol oder Ethylbenzol, oder Amide wie Formamid, Dimethylformamid oder N-Methylpyrrolidon.

Bevorzugt wird die erfindungsgemäße Umsetzung ohne Lösungsmittel in Substanz durchgeführt.

Bei einem kontinuierlich betriebenen Reaktor (R1) werden die Edukte bevorzugt in flüssigem Zustand in den Reaktor gepumpt. Der Strom der Einsatzstoffe in den Reaktor kann von oben nach unten (Rieselfahrweise) oder von unten nach oben (Sumpffahrweise) erfolgen.

### Zufuhr von Wasserstoff

Dem Reaktor (R1), in dem die Umsetzung der Dinitrile, Trinitrile und/oder Aminonitrile der Formel (la), (IIa), (Ib), (Ib'), (IIb) zu Polyaminen erfolgt, wird Wasserstoff zugeführt. In dem erfindungsgemäßen Verfahren besitzt Wasserstoff eine Reihe von vorteilhaften Eigenschaften. Zunächst einmal dient Wasserstoff zur Hydrierung der Nitrilgruppen zu Aminogruppen. Weiterhin hält der Wasserstoff den Hydrierkatalysator aktiv.

In einer bevorzugten Ausführungsform der erfindungsgemäßen diskontinuierlichen und kontinuierlichen Umsetzung lassen sich der Umsatz der eingesetzten Dinitrile, Trinitrile und/oder Aminonitrile der Formel (la), (IIa), (Ib), (Ib'), (IIb), die Selektivität und das mittlere Molgewicht der erhaltenen Polyamine dadurch deutlich steigern, dass man bei konstantem Druck überschüssigen Wasserstoff durch den ersten Reaktor (R1) leitet. Unter überschüssigem Wasserstoff ist dabei zu verstehen, dass die zugeführte Wasserstoff-Menge deutlich grösser als die für die Nitrilgruppen-Hydrierung notwendige Menge ist (siehe Beispiele 1a bis 1c). Der Überschuss der Molmenge an Wasserstoff, bezogen auf die theoretisch zur Hydrierung der Nitrilgruppen notwendigen Wasserstoff-Menge, beträgt 0,1 bis 20 Mole, bevorzugt 1 bis 10 Mole, besonders bevorzugt 1 bis 5 Mole.
Durch den strömenden Wasserstoff wird der bei der Polykondensation freigesetzte Ammoniak aus dem Reaktor entfernt.

Der verwendete Wasserstoff kommt im Allgemeinen technisch rein zum Einsatz. Der Wasserstoff kann auch in Form eines Wasserstoff enthaltenen Gases, das heißt mit Beimengungen anderer Inertgase, wie Stickstoff, Helium, Neon, Argon oder Kohlendioxid verwendet werden. Als Wasserstoff enthaltende Gase können beispielsweise Reformerabgase, Raffineriegase usw. verwendet werden, sofern diese Gase keine Kontaktgifte für die eingesetzten Katalysatoren, wie z.B. Kohlenmonoxid, enthalten. Bevorzugt wird jedoch reiner Wasserstoff bzw. im Wesentlichen reiner Wasserstoff in das Verfahren eingesetzt, beispielsweise mit einem Gehalt an Wasserstoff von mehr als 99 Gew.-%, bevorzugt mehr als 99,9% Wasserstoff, besonders bevorzugt von mehr als 99,99%, insbesondere mehr als 99,999%.

Durch Variation der Wasserstoff-Menge, die den Reaktor durchströmt, kann die gesamte freiwerdende Ammoniak-Menge oder nur ein Teil dieser Ammoniak-Menge aus dem Reaktor entfernt werden.

Anstelle von Wasserstoff können auch Gemische aus Wasserstoff und einem oder mehreren Inertgasen verwendet werden. Wasserstoff oder Gemische aus Wasserstoff und einem Inertgas werden in die Flüssigphase oder die Gasphase, bevorzugt in die Flüssigphase eingeleitet.

Aus den beim Durchleiten von Wasserstoff oder Gemischen aus Wasserstoff und Inertgas erhaltenen Gasgemischen wird der Ammoniak durch Kühlung oder Destillation abgetrennt und ganz überwiegend aus dem Verfahren ausgeschleust. Da Nitrilhydrierungen jedoch vorteilhaft in Gegenwart von basischen Verbindungen wie Ammoniak oder Alkalihydroxiden durchgeführt werden (Vermeidung der Bildung von tertiären Aminen), kann gegebenen falls auch eine Teilmenge des Ammoniaks in den Reaktor (R1) zurückgeführt werden. Nicht verbrauchter Wasserstoff oder Gemische aus Wasserstoff und Inertgas werden ganz oder teilweise in den ersten oder zweiten, bevorzugt in den ersten Reaktor (R1) zurückgeführt.

Als Inertgase werden im folgenden Gase bezeichnet, die sich unter den vorliegenden Reaktionsbedingungen überwiegend inert verhalten und im Wesentlichen nicht mit den im Reaktionsgemisch vorhandenen Verbindungen reagieren. Als Inertgase werden bevorzugt Stickstoff oder die Edelgase Helium, Neon, Argon oder Xenon eingesetzt. Ganz besonders bevorzugt wird Stickstoff verwendet. Als Inertgase können auch Gemische aus den voranstehend genannten Gasen eingesetzt werden.

### Reaktor (R1)

Die Herstellung der Polyamine in Gegenwart der genannten Katalysatoren wird bevorzugt in den üblichen für die Katalyse geeigneten Reaktionsgefäßen in Festbett- oder Suspensionsfahrweise kontinuierlich, diskontinuierlich oder semikontinuierlich in einem Reaktor (R1) oder zwei Reaktoren (R1) und (R2), die in Serie geschaltet sind, durchgeführt.

### Festbett

In einer besonders bevorzugten Ausführungsform wird die erfindungsgemäße Umsetzung in einem Reaktor durchgeführt, in dem der Katalysator als Festbett angeordnet ist. Geeignete Festbettreaktoren sind beispielsweise in dem Artikel "Catalytic Fixed-Bed Reactors" in Ullmanns Encyclopedia of Industrial Chemistry, Published Online: 15 JUN 2000, DOI: 10.1002/14356007.b04-199 beschrieben.

Bevorzugt wird das Verfahren in einem Schachtreaktor, einem Rohrbündelreaktor oder besonders bevorzugt in einem Rohrreaktor durchgeführt.

Rohrreaktoren können jeweils als einzelne Reaktoren, als Serie von einzelnen Reaktoren und/oder in Form von zwei oder mehr parallelen Reaktoren eingesetzt werden. Bevorzugt ist, mit zwei Reaktoren, besonders bevorzugt mit einem Reaktor zu arbeiten. Wird nur ein Reaktor verwendet, kann es vorteilhaft sein, den Reaktor in zwei Temperaturzonen aufzuteilen. Dabei findet die Nitrilhydrierung im Bereich der tieferen, die Polykondensation im Bereich der höheren Temperatur statt.

### Suspension

In einer bevorzugten Ausführungsform wird der Katalysator im zu polymerisierenden Reaktionsgemisch suspendiert. Die Polymerisation in Suspensionsfahrweise kann bevorzugt in einem Rührreaktor, Strahlschlaufenreaktor, Strahldüsenreaktor, Blasensäulenreaktor oder in einer Kaskade derartiger gleicher oder verschiedener Reaktoren durchgeführt werden.

Besonders bevorzugt wird die Polykondensation in Suspensionsfahrweise in einem Rührreaktor durchgeführt.

Im Gegensatz zur Polykondensation von Diaminen handelt es sich ausgehend von Dinitrilen, Trinitrilen und/oder Aminonitrilen der Formel (la), (IIa), (Ib), (Ib'), (IIb), deren Nitrilgruppen zunächst hydriert werden, um stark exotherme Reaktionen. Daher müssen in den Reaktoren und/oder in Produktkreisströmen Kühlvorrichtungen vorgesehen werden.

### Aufarbeitung der Polyamine

### Aufarbeitung - Kolonne K1

In einer ganz besonders bevorzugten Ausführungsform wird der Reaktionsaustrag in eine Destillationskolonne entspannt.
Die Kolonne wird im Allgemeinen so betrieben, dass Ammoniak und zugeführtes Gas am Kopf der Kolonne abgezogen werden und die restliche Flüssigphase (Monomer, Oligomere und Polymere) am Sumpf der Kolonne abgezogen werden (Variante 1).

Die Kolonne K1 kann aber auch so betrieben werden, dass am Kopf Ammoniak und das zugeführte Gas abgezogen werden, aus einem Abzug im mittleren Bereich der Kolonne monomeres und oligomeres Dinitril/Trinitril/Aminonitril der Formel (I) und deren entsprechenden Diamine abgezogen wird und höhermolekulares Polyamin am Sumpf der Kolonnen abgezogen wird (Variante 2).

Die genauen Betriebsbedingungen der Destillationskolonne können entsprechend der Trennleistung der verwendeten Kolonne vom Fachmann anhand der bekannten Dampfdrucke und Verdampfungsgleichgewichte der in die Destillationskolonne eingeleiteten Komponenten nach herkömmlichen Berechnungsmethoden routinemäßig ermittelt werden.

### Variante 1

Der Reaktoraustrag wird vorzugsweise in den mittleren Bereich einer Destillationskolonne K1 entspannt.
Die Destillationskolonne K1 wird besonders bevorzugt in einer Bodenkolonne durchgeführt. Bei einer Bodenkolonne befinden sich im Inneren der Kolonne Zwischenböden, auf denen der Stoffaustausch stattfindet. Beispiele für unterschiedliche Bodentypen sind Siebböden, Tunnelböden, Dualflowböden, Glockenböden oder Ventilböden.

In einer weiteren bevorzugten Ausführungsform können die destillativen Einbauten als geordnete Packung vorliegen, beispielsweise als Blechpackung, wie Mellapak 250 Y oder Montz Pak, Typ B1-250, oder als strukturierte Keramikpackung oder als ungeordnete Packung, z.B. aus Pallringen, IMTP-Ringen (Fa. Koch-Glitsch), Raschig-Superringen, etc.
Am Kopf der Kolonne K1 wird in der Regel ein gasförmiger Strom aus dem zugeführten Gas und Ammoniak erhalten.

In einer besonders bevorzugten Ausführungsform wird aus dem am Kopf anfallenden Gasstrom Ammoniak abgetrennt. Die Abtrennung von Ammoniak aus dem ausgeschleusten Gasstrom kann bevorzugt dadurch erfolgen, dass der Gasstrom durch eine Kühlvorrichtung auf eine Temperatur gekühlt wird, bei dem Ammoniak in den flüssigen Zustand übergeht, und das zugeführte Gas in der Gasphase verbleibt. Die Kühlvorrichtung ist bevorzugt ein Kondensator.

Der Kondensator der Destillationskolonne K1 wird im Allgemeinen bei einer Temperatur betrieben, in dem das Ammoniak bei dem entsprechenden Kopfdruck weitestgehend vollständig kondensiert wird.

Der kondensierte Ammoniak wird bevorzugt aus dem Verfahren ausgeschleust.

Das nicht kondensierte Gas, welches im Wesentlichen aus Inertgas und/oder Wasserstoff besteht, wird bevorzugt in das Verfahren zurückgeführt. Bevorzugt ist das zurückgeführte Gas im Wesentlichen frei von Ammoniak.

Die Kolonne K1 erfordert in der Regel keinen zusätzlichen Verdampfer am Sumpf der Kolonne, da die Differenz zwischen den Siedepunkten von Ammoniak und monomerem Dinitril/Trinitril/Aminonitril der Formel (I) und deren entsprechenden Diamine in der Regel so hoch ist, dass eine gute Trennung von Ammoniak und monomeren Dinitril/Trinitril/Aminonitril der Formel (I) und deren entsprechenden Diamine ohne zusätzliche Sumpfbeheizung gelingt.

Es ist jedoch auch möglich den Sumpf der Kolonne zu beheizen, beispielsweise mit einem Sumpfverdampfer.
Die Temperatur im Sumpf der Kolonne sollte dann so eingestellt, dass bei dem in der Kolonne herrschenden Kopfdruck Ammoniak weitestgehend vollständig verdampft, während monomeres Diamin in der flüssigen Phase verbleibt.

Der Sumpfaustrag der Kolonne K1 enthält im Wesentlichen Dinitril/Trinitril/Aminonitril der Formel (I) und deren entsprechenden Diamine , Polyamine und ggf. Lösungsmittel.

### Ein Teil des Sumpfaustrags kann

a) dem Reaktor zurückgeführt werden, oder
b) in eine weitere Kolonne K2 eingeleitet werden, in der monomeres Dinitril/Trinitril/Aminonitril der Formel (I) und deren entsprechenden Diamine und leichtsiedendes Oligomer von höhersiedendem Polyamin getrennt wird, oder
c) dem Reaktor als Reaktionsprodukt entnommen werden.
d) Ein Teil des Sumpfaustrags aus der Kolonne K1 kann in den Reaktor zurückgeführt werden, wo eine weitere Kondensation stattfindet. So können Polymere mit einem besonders hohen Molekulargewicht erzielt werden.

Es ist bevorzugt, dass der zurückgeführte Sumpfaustrag im Wesentlichen kein Ammoniak enthält. Dies wird im Allgemeinen bereits nach der Entspannungsverdampfung (Destillation) erreicht. Sollten die Ammoniakgehalte dennoch höher sein, so kann der Ammoniakgehalt verringert werden, beispielsweise durch Destillation oder Entgasung (Strippung).
b) Der Sumpfaustrag aus der Kolonne K1 kann in eine weitere Destillationskolonne K2 eingeleitet werden, die so betrieben wird, dass am Kopf der Kolonne monomeres Dinitril/Trinitril/Aminonitril der Formel (I) und deren entsprechenden Diamine und leichtsiedendes Oligopolyamin anfällt und am Sumpf der Kolonne Polyamin abgezogen wird. Die Kolonne K2 wird nachfolgend näher beschrieben.
c) Ein Teil des Sumpfproduktes aus der Kolonne K1 kann als Reaktionsprodukt aus dem Verfahren ausgeschleust werden.

### Variante 2

Die Kolonne K1 kann auch so betrieben werden, dass am Kopf der Kolonne Ammoniak und das zugeführte Gas anfallen, im mittleren Bereich eine Fraktion als Seitenabzug entnommen wird, die monomeres Dinitril/Trinitril/Aminonitril der Formel (I) und deren entsprechenden Diamine und nieder siedende Oligomere enthält und am Sumpf der Kolonne K1 Polyamin anfällt.

Der Reaktoraustrag wird, wie bei der zuvor beschriebenen Variante 1, vorzugsweise in den mittleren Bereich, einer wie zuvor beschriebenen Destillationskolonne K1 entspannt.

Am Kopf der Kolonne K1 wird in der Regel ein gasförmiger Strom aus dem zugeführten Gas und Ammoniak erhalten.

In einer besonders bevorzugten Ausführungsform wird aus dem am Kopf anfallenden Gasstrom Ammoniak abgetrennt. Die Abtrennung von Ammoniak aus dem ausgeschleusten Gasstrom kann bevorzugt dadurch erfolgen, dass der Gasstrom durch eine Kühlvorrichtung auf eine Temperatur gekühlt wird, bei dem Ammoniak in den flüssigen Zustand übergeht, und das zugeführte Gas in der Gasphase verbleibt. Die Kühlvorrichtung ist bevorzugt ein Kondensator.

Der Kondensator der Destillationskolonne K1 wird im Allgemeinen bei einer Temperatur betrieben, in dem Ammoniak bei dem entsprechenden Kopfdruck weitestgehend vollständig kondensiert wird.

Der kondensierte Ammoniak wird bevorzugt aus dem Verfahren ausgeschleust.
Das nicht kondensierte Gas, welches im Wesentlichen aus Inertgas und/oder Wasserstoff besteht, wird bevorzugt in das Verfahren zurückgeführt.

Die Kolonne K1 erfordert in der Regel keinen zusätzlichen Verdampfer am Sumpf der Kolonne, da die Differenz zwischen den Siedepunkten von Ammoniak und monomerem Dinitril/Trinitril/Aminonitril der Formel (I) und deren entsprechenden Diamine in der Regel so hoch ist, dass eine gute Trennung von Ammoniak und monomeren Dinitril/Trinitril/Aminonitril der Formel (I) und deren entsprechenden Diamine ohne zusätzliche Sumpfbeheizung gelingt.
Es ist jedoch auch möglich den Sumpf der Kolonne zu beheizen, beispielsweise mit einem Sumpfverdampfer.

Die Temperatur im Sumpf der Kolonne sollte so eingestellt, dass bei dem in der Kolonne herrschenden Kopfdruck Ammoniak weitestgehend vollständig verdampft, während monomeres Dinitril/Trinitril/Aminonitril der Formel (I) und deren entsprechenden Diamine in der flüssigen Phase verbleibt.

Als Seitenabzug der Kolonne K1 wird bevorzugt eine Fraktion abgezogen, die im Wesentlichen Oligomere des Dinitrils/Trinitrils/Aminonitrils der Formel (I) und deren entsprechenden Diamine enthält.

### Der Seitenabzug kann

a) aus dem Verfahren ausgeschleust werden, oder
b) in das Verfahren zurückgeführt werden (bevorzugte Variante).

Wenn der Seitenabzug in das Verfahren zurückgeführt wird, dann ist es bevorzugt, dass der Seitenabzug im Wesentlichen kein Ammoniak enthält. Dies wird im Allgemeinen bereits nach der Entspannungsverdampfung (Destillation) erreicht. Sollten die Ammoniakgehalte dennoch höher sein, so kann der Ammoniakgehalt verringert werden, beispielsweise durch Destillation oder Entgasung (Strippung).

Der Sumpfaustrag der Kolonne K1 enthält im Wesentlichen Dinitril/Trinitril/Aminonitril der Formel (I) und deren entsprechenden Diamine , Oligomere des Dinitrils/Trinitril/Aminonitrils der Formel (I) und deren entsprechenden Diamine , Polyamin und ggf. Lösungsmittel.

Ein Teil des Sumpfaustrags kann, wie unter Variante 1 beschrieben
a) dem Reaktor zurückgeführt werden, oder
b) in eine weitere Kolonne K2 eingeleitet werden, in der monomeres Dinitril/Trinitril/Aminonitril der Formel (I) und deren entsprechenden Diamine und leichtsiedendes Oligomer von höhersiedendem Polyamin getrennt wird, oder
c) dem Reaktor als Reaktionsprodukt entnommen werden.

### Aufarbeitung - Kolonne K2

Der Sumpfaustrag aus Kolonne K1 kann in eine weitere Kolonne K2 eingeleitet werden, die so betrieben wird, dass am Kopf der Kolonne monomeres Dinitril/Trinitril/Aminonitril der Formel (I) und deren entsprechenden Diamine und leichtsiedende Oligomere anfällt. Die Kolonne K2 kann aber auch so betrieben werden, dass am Kopf überwiegend monomeres Dinitril/Trinitril/Aminonitril der Formel (I) und deren entsprechenden Diamine an einem Seitenabzug überwiegend oligomeres Dinitril/Trinitril/Aminonitril der Formel (I) und deren entsprechenden Diamine und am Sumpf polymeres Dinitril/Trinitril/Aminonitril der Formel (I) und deren entsprechenden Diamine abgezogen werden kann.

Der Sumpfaustrag aus Kolonne K1 wird vorzugsweise in den mittleren Bereich, einer Destillationskolonne K2 zugeführt.

Bevorzugt weist die Destillationskolonne K2 Einbauten zur Erhöhung der Trennleistung auf. Die destillativen Einbauten können beispielsweise als geordnete Packung vorliegen, beispielsweise als Blechpackung wie Mellapak 250 Y oder Montz Pak, Typ B1-250. Es kann auch eine Packung mit geringerer oder erhöhter spezifischer Oberfläche vorliegen, oder es kann eine Gewebepackung oder eine Packung mit anderer Geometrie wie Mellapak 252 Y verwendet werden. Vorteilhaft bei der Verwendung dieser destillativen Einbauten sind der geringe Druckverlust und der geringe spezifische Flüssig-Hold-up im Vergleich zu beispielsweise Ventilböden. Die Einbauten können in einen oder mehreren Betten vorliegen.

Der Sumpf der Kolonne K2 ist vorzugsweise mit einem Sumpfverdampfer ausgestattet. Die Temperatur im Sumpf der Kolonne sollte so eingestellt, dass bei dem in der Kolonne herrschenden Kopfdruck Ammoniak, monomeres Dinitril/Trinitril/Aminonitril der Formel (I) und deren entsprechenden Diamine weitestgehend vollständig und ein Teil der Oligomeren verdampft, während polymeres Polyamin in der flüssigen Phase verbleibt.

Am Kopf der Kolonne K2 wird in der Regel ein gasförmiger Strom abgezogen, der im Wesentlichen aus monomerem Dinitril/Trinitril/Aminonitril der Formel (I) und deren entsprechenden Diamine besteht.
Bevorzugt wird der am Kopf anfallende Gasstrom einem Kondensator zugeführt, der mit der Destillationskolonne K2 verbunden ist.

Der Kondensator der Destillationskolonne K2 wird im Allgemeinen bei einer Temperatur betrieben, in dem das Dinitril/Trinitril/Aminonitril der Formel (I) und deren entsprechenden Diamine bei dem entsprechenden Kopfdruck weitestgehend vollständig kondensiert wird.

Das Kondensat der Kolonne K2, welches im Wesentlichen aus monomerem Dinitril/Trinitril/Aminonitril der Formel (I) und deren entsprechenden Diaminen besteht, kann ausgeschleust werden oder in das Verfahren zurückgeführt werden.
Das zurückgeführte Dinitril/Trinitril/Aminonitril der Formel (I) und deren entsprechenden Diamine enthält vorzugsweise im Wesentlichen kein Ammoniak.
Dies hat den Vorteil, dass Polyamine mit hohem Molekulargewicht und geringen Verzweigungsgraden erhalten werden können. Weiterhin kann die Reaktionszeit bis zum Erreichen eines gewissen Umsetzungsgrads verringert werden (Erhöhung der Reaktionsgeschwindigkeit). Sollte der Ammoniakgehalt höher sein, so kann das Diamin einer weiteren Destillation oder Entgasung, beispielsweise einer Strippung, unterzogen werden.

Ein Teil des als Kondensat anfallenden Dinitrils/Trinitrils/Aminonitrils der Formel (I) und deren entsprechenden Diamine kann als Rücklauf in die Kolonne zurückgeführt werden.

Ein Teil des Sumpfaustrags kann dem Reaktor zurückgeführt werden, oder dem Reaktor als Reaktionsprodukt entnommen werden. Bevorzugt wird das Sumpfprodukt der Kolonne K2 als Reaktionsprodukt ausgeschleust.

In der Kolonne K2 kann auch ein Seitenabzug entnommen werden, der eine Fraktion aus leichtsiedenden Oligomeren enthält. Diese Oligomere können ausgeschleust werden, oder zusammen mit dem am Kopf ausgeschleusten Dinitril/Trinitril/Aminonitril der Formel (I) und deren entsprechenden Diamine in den Reaktor zurückgeführt werden.

### Bevorzugte Verfahrensvarianten

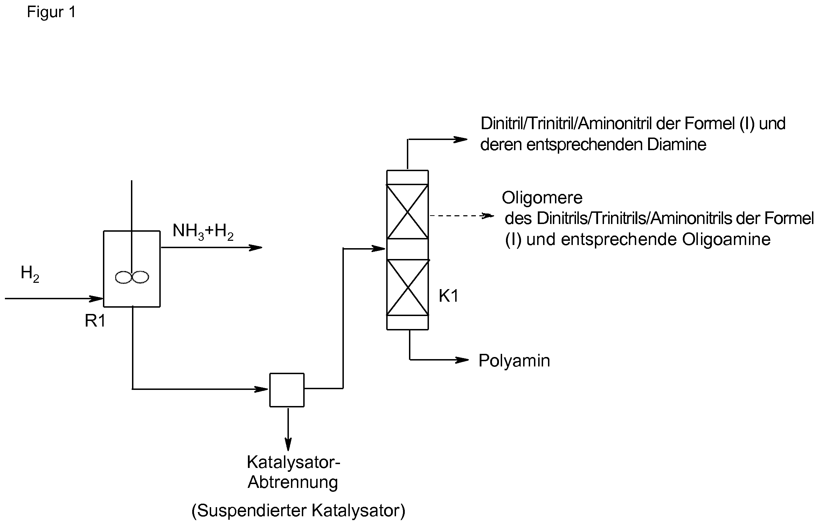

In den Figuren 1 bis 6 werden besondere Ausführungsformen des erfindungsgemäßen Verfahrens beschrieben.

### Variante D-1

In Figur 1 ist ein diskontinuierliches Verfahren dargestellt, bei dem Monomer in einem Rührkesselreaktor R 1 vorgelegt wird, welcher den Katalysator in suspendierter oder fest angeordneter Form, z.B. in einem Metallnetz enthält. Dann wird kontinuierlich Inertgas und/oder Wasserstoff zugeleitet. Die Einleitung erfolgt bevorzugt durch ein Gaseinleitungsrohr, einen Gasverteilerring oder eine Düse, welches bevorzugt unterhalb eines Rührers angeordnet ist. Der eingeleitete Gasstrom wird durch den Energieeintrag des Rührers in kleine Gasblasen zerschlagen und im Reaktor homogen verteilt. Ein Gemisch aus gebildetem Ammoniak und Inertgas und/oder Wasserstoff wird kontinuierlich durch eine Auslassöffnung im oberen Bereich des Reaktors aus dem Reaktor ausgeschleust.

Wird die diskontinuierliche Hydrierung und Polykondensation nicht in Gegenwart eines fest angeordneten, sondern eines suspendierten Katalysators durchgeführt, so wird im Rahmen der Wertprodukt-Aufarbeitung beim Auslassen des Produkts zunächst der Suspensionskatalysator abgetrennt, z.B. durch Filtration oder Zentrifugieren.

Der bei der diskontinuierlichen Polykondensation erhaltene Reaktionsaustrag kann in eine Destillationskolonne K1 geleitet werden, in der am Kopf ein Strom aus Dinitril/Trinitril/Aminonitril der Formel (I) und deren entsprechenden Diamine und Oligomeren des Dinitrils/Trinitrils/Aminonitrils der Formel (I) und deren entsprechenden Diamine abgetrennt wird. Im Sumpf der Kolonne wird Polyamin erhalten.

Der bei der diskontinuierlichen Polykondensation erhaltene Reaktionsaustrag kann alternativ in eine Destillationskolonne K1 geleitet werden, in der am Kopf ein Strom aus Dinitril/Trinitril/Aminonitril der Formel (I) und deren entsprechenden Diamine und als Seitenabzug eine Fraktion, die im Wesentlichen aus Oligomeren des Dinitrils/Trinitrils/Aminonitrils der Formel (I) und deren entsprechenden Diamine besteht abgetrennt wird. Im Sumpf der Kolonne wird Polyamin abgezogen.

### Variante D-2

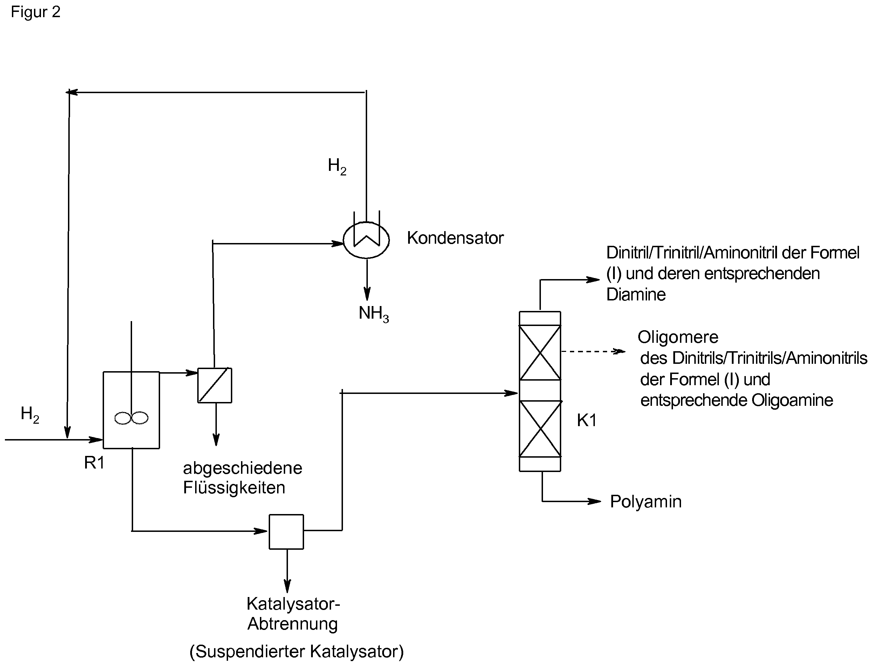

In Figur 2 wird eine Variante des Verfahrens dargestellt, in der der ausgeschleuste Gasstrom nach der Ausschleusung entspannt wird. Zur Abtrennung von mitgerissener Flüssigkeit wird der abgezogene Gasstrom in einen Flüssigkeitsabscheider eingeleitet. Die im Flüssigkeitsabscheider abgeschiedene Flüssigkeit wird aus dem Verfahren ausgeschleust. Nach dem Flüssigkeitsabscheider wird bevorzugt das aus dem Reaktor ausgeschleuste Gemisch aus Ammoniak und Inertgas und/oder Wasserstoff gekühlt, wobei der Ammoniak verflüssigt wird und vom Inertgas und/oder Wasserstoff abgetrennt werden kann. Das Inertgas und/oder Wasserstoff kann wieder komprimiert, wenn notwendig mit Frisch-Inertgas und/oder Wasserstoff versetzt und in die Polymerisationsstufe zurückgeführt werden.

### Variante D-3

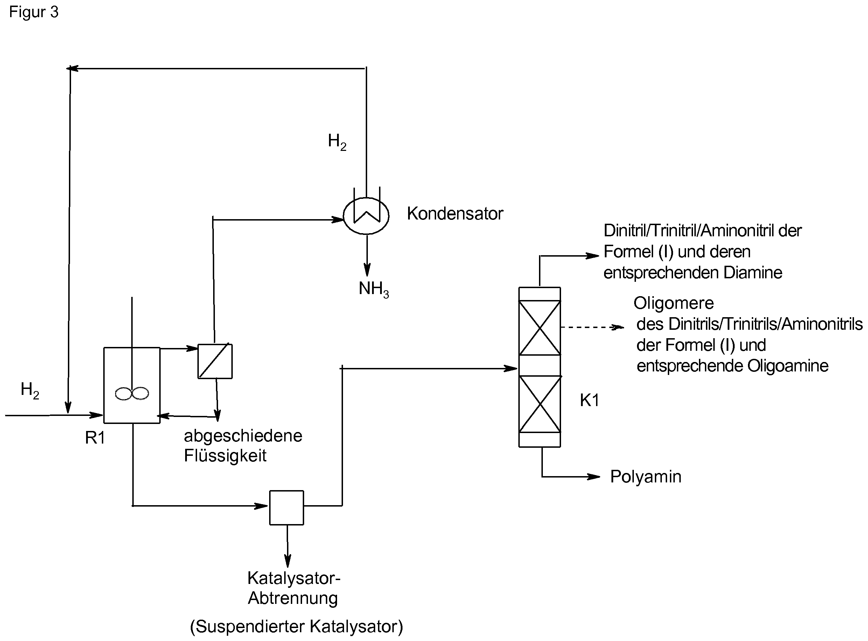

In Figur 3 wird eine weitere Variante dargestellt, in der die in dem Flüssigkeitsabscheider abgeschiedene Flüssigkeit, die im Wesentlichen aus Dinitril/Trinitril/Aminonitril der Formel (I) und deren entsprechenden Diamine , Oligomeren des Dinitrils/Trinitrils/Aminonitrils der Formel (I) und deren entsprechenden Diamine und ggf. Lösungsmittel besteht, in das Verfahren zurückgeführt wird. Sollte das Gemisch aus Dinitril/Trinitril/Aminonitril der Formel (I) und deren entsprechenden Diamine und/oder Oligomeren der Dinitrile/Trinitrile/Aminonitrile der Formel (I) und deren entsprechenden Diamine Nebenprodukte enthalten, so können diese, beispielsweise durch Destillation, von den Dinitrilen/Trinitrilen/Aminonitrilen der Formel (I) und deren entsprechenden Diamine und deren Oligomeren vor ihrer Rückführung abgetrennt werden.

### Variante K-1

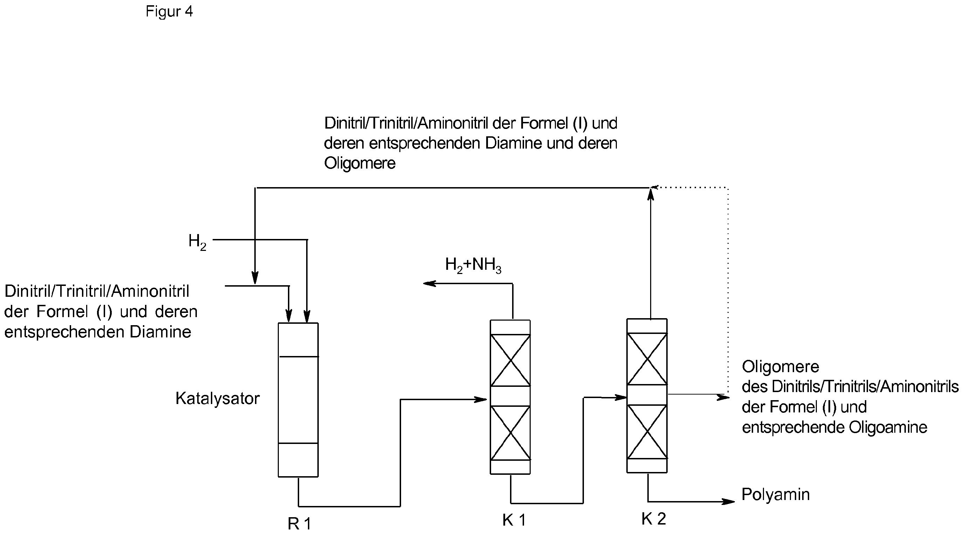

In Figur 4 ist ein kontinuierliches Verfahren zur Herstellung von Polyaminen dargestellt. Dinitril/Trinitril/Aminonitril der Formel (I) und deren entsprechenden Diamine wird zusammen mit Inertgas und/oder Wasserstoff über einen Katalysator geleitet, der in einem inertisierten Druckreaktor R1 fest angeordnet ist.

Der Reaktionsaustrag wird auf eine Kolonne K1 geleitet. Über Kopf der Kolonne K1 geht ein Gemisch aus Ammoniak und Wasserstoff, welches aus dem Verfahren ausgeschleust wird. Das Sumpfprodukt der Kolonne K1 wird auf eine Kolonne K2 geführt. Über Kopf der Kolonne K2 wird nicht umgesetztes Dinitril/Trinitril/Aminonitril der Formel (I) und deren entsprechenden Diamine abgetrennt und in den Reaktor R1 zurückgeführt. Aus einem Seitenabzug der Kolonne K2 werden ggf. Oligomere abgezogen, die ausgeschleust und /oder in den Reaktor R1 zurückgeführt werden. Das Sumpfprodukt der Kolonne K2 enthält Polyamin.

### Variante K-2

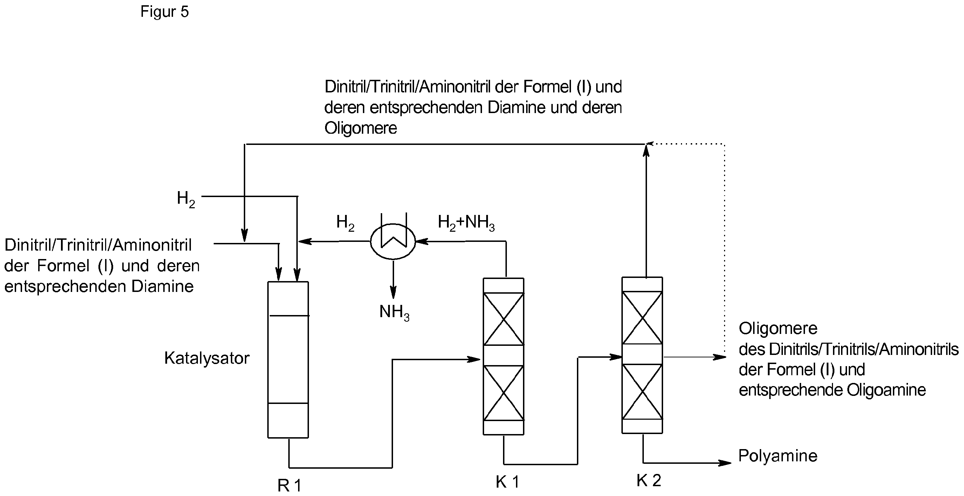

In Figur 5 ist ein kontinuierliches Verfahren zur Herstellung von Polyaminen dargestellt. Dinitril/Trinitril/Aminonitril der Formel (I) und deren entsprechenden Diamine wird zusammen mit Inertgas und/oder Wasserstoff über einen Katalysator geleitet, der in einem inertisierten Druckreaktor R1 fest angeordnet ist.

Der Reaktionsaustrag wird auf eine Kolonne K1 geleitet. Über Kopf der Kolonne K1 geht ein Gemisch aus Ammoniak und Wasserstoff, aus dem der Ammoniak auskondensiert wird. Inertgas und/oder Wasserstoff können in den Reaktor R1 zurückgeführt werden.

Das Sumpfprodukt der Kolonne K1 wird auf eine Kolonne K2 geführt. Über Kopf der Kolonne K2 wird nicht umgesetztes Dinitril/Trinitril/Aminonitril der Formel (I) und deren entsprechenden Diamine und niedersiedendes Oligomer abgetrennt und in den Reaktor R1 zurückgeführt. Aus einem Seitenabzug der Kolonne K2 werden ggf. Oligomere abgezogen, die ausgeschleust und/oder in den Reaktor R1 zurückgeführt werden. Das Sumpfprodukt der Kolonne K2 enthält Polyamin.

### Variante K-3

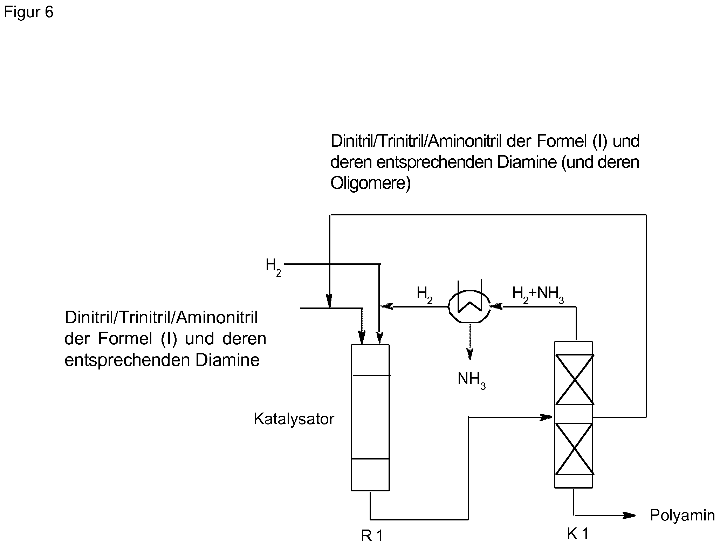

In Figur 6 ist eine Variante des kontinuierlichen Verfahrens dargestellt.

Dinitril/Trinitril/Aminonitril der Formel (I) und deren entsprechenden Diamine wird zusammen mit Inertgas und/oder Wasserstoff über einen Katalysator geleitet, der in einem inertisierten Druckreaktor R1 fest angeordnet ist. Unter den Reaktionsbedingungen entsteht ein Reaktionsaustrag, der auf eine Kolonne K1 geleitet wird. Die Kolonne K1 wird so betrieben, dass als Kopfprodukt ein Gemisch aus Ammoniak sowie Inertgas und/oder Wasserstoffgemisch erhalten wird, aus einem Seitenabzug ein Gemisch aus Dinitril/Trinitril/Aminonitril der Formel (I) und deren entsprechenden Diamine und Oligomeren des Dinitrils/Trinitril/Aminonitrils der Formel (I) und deren entsprechenden Diamine und als Sumpfprodukt Polyamin entnommen wird. Die Kolonne K2 in Figur 4 oder 5 entfällt.

Gegebenenfalls nicht umgesetztes Dinitril/Trinitril/Aminonitril der Formel (I) und deren entsprechenden Diamine und oligomere Polyamine können destillativ abgetrennt und in die Polyamin-Synthesestufe zurückgeführt werden.

### 6. Ausführungsbeispiele

### 6.1. Einstufige Herstellung von Polyhexamethylenpolyamin aus Adiponitril (batch-Fahrweise)

Die Experimente wurden in einem mit einem Blattrührer gerührten 300 ml Druckgefäß aus Stahl durchgeführt. Über ein Einleitungsrohr wurde Wasserstoff zugeführt. Im oberen Teil des Druckgefäßes konnte optional Abgas abgeleitet werden, das ohne Kühlung in die Mitte eines senkrecht stehenden Stahlrohrs (Innendurchmesser 1,4 cm, Höhe 16 cm) geführt wurde. Hier anfallendes flüssiges Kondensat wurde in den unteren Teil des Druckgefäßes zurückgeführt, Abgas über das Stahlrohr aus der Apparatur abgeleitet.

Als Katalysator wurde ein Kobaltkatalysator mit einem Strangdurchmesser von 4 mm eingesetzt. Seine Herstellung ist in EP-A-0636409 beschrieben. Die Katalysator-Formkörper wurden bei 280°C und Normaldruck 12 Stunden lang durch einen kontinuierlichen Wasserstoffstrom von 50 NI pro Stunde reduziert. Als Einsatzstoff wurden 50 g Adiponitril (ADN) unter Stickstoff im Druckgefäß vorgelegt. 50 g des aktivierten Katalysators wurden in einem "Metallkäfig" fixiert, der von dem gerührten Reaktionsgemisch durchströmt wurde. Die Polykondensation erfolgte 4 Stunden lang bei 160°C und 60 bar Gesamtdruck. Nach der Reaktionszeit wurde der Autoklav auf Raumtemperatur abgekühlt und entspannt. Das Reaktionsgemisch wurde aus dem Autoklaven entnommen. Die Reaktionsausträge wurden gaschromatographisch (Gewichts-%) und durch Gelpermeationschromatographie (Absoluteichung durch Messung von definierten Polyamin-Standards) analysiert.

### Beispiel 1a

Der Versuch wurde wie oben beschrieben durchgeführt. In dem Druckgefäß wurde während der gesamten Versuchszeit durch Aufpressen von Wasserstoff ein Gesamtdruck von 60 bar aufrechterhalten. Es wurde kein Abgas aus dem Druckgefäß abgeleitet. Die Analyse des Reaktionsaustrags (Tabelle 1) zeigt, dass nur 11% Polyamine (Hochsieder) entstanden waren.

### Beispiel 1b

Der Versuch wurde wie oben beschrieben durchgeführt. Während der vier Stunden Reaktionszeit wurden jedoch kontinuierlich bei einem Druck von 60 bar pro Stunde 2,4 Mole Wasserstoff pro Mol ADN durch das Druckgefäß geleitet und entsorgt. Flüssiges Kondensat wurde in das Druckgefäß zurückgeführt. Die Strukturen der Nebenprodukte Hexamethylenimin (HMI), Aminohexyl-HMI, HMI-HMD-HMI, Bis-HMD und HMI-(HMD)₂ sind in Abbildung 1 wiedergegeben.

**Tabelle 1**

| Bsp. | T [°C] | HMI | HMD | Aminohexyl-HMI | Bis-HMD | HMI-HMD-HMI | HMI-(HMD)2 | Sonstige | Hochsieder | M_{w} | PDI |
|---|---|---|---|---|---|---|---|---|---|---|---|
| 1a | 160 | 13 | 47 | 1 | 23 | - | - | 5 | 11 | - | - |
| 1b | 160 | 9 | 2 | 6 | 9 | 1 | 4 | 7 | 62 | 750 | 1,6 |

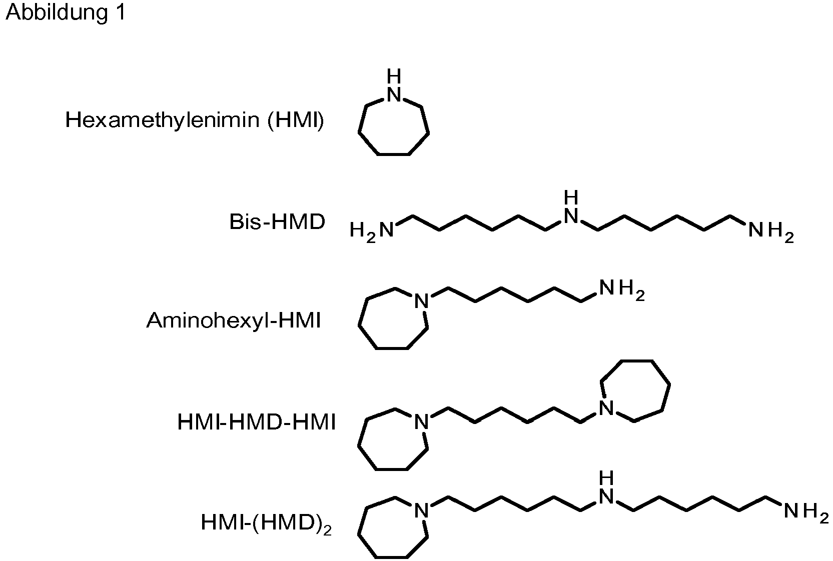

Die Analyse des Reaktionsaustrages (Tabelle 1) demonstriert, dass das Durchleiten von Wasserstoff unter Entfernung von gebildetem Ammoniak den Umsatz beträchtlich erhöht und zu mittleren Molgewichten von 750 g/mol führt.

### 6.2. Einstufige Herstellung von Polyhexamethylenpolyamin aus Adiponitril (kontinuierliche Fahrweise)

### Beispiel 1c

Adiponitril wurde kontinuierlich von unten nach oben über einen Katalysator geleitet, der in einem Rohrreaktor enthalten war. Als Katalysator wurden 378g aktivierter Kobaltkatalysator mit einem Strangdurchmesser von 4 mm eingesetzt, dessen Herstellung in der EP-A-0636409 als Katalysator A auf Seite 3, Zeile 35-44 beschrieben ist. Der Druck betrug 50 bar, die Temperatur 170°C. Die Katalysatorbelastung lag bei 0,1 kg/Lh Adiponitril. Es wurden 5 Mole Wasserstoff pro Mol Adiponitril durch den Reaktor geleitet (Abgasfahrweise). Der Rohaustrag war ein weißer, wachsartiger Feststoff. Die Zusammensetzung des Rohaustrags ist in Tabelle 2 zusammengefasst. Die Molgewichtsbestimmung erfolgte nach destillativer Abtrennung von HMI, HMD, Aminohexyl-HMI und Bis-HMD.

**Tabelle 2**

| T [°C] | HMI | HMD | Aminohexyl-HMI | Bis-HMD | HMI-HMD-HMI | HMI-(HMD)2 | Sonstige | Hochsieder | M_{w} | PDI |
|---|---|---|---|---|---|---|---|---|---|---|
| 170 | 1 | 0 | 2 | 0 | 9 | 1 | 14 | 73 | 1996 | 1,7 |

### 6.3. Einstufige Herstellung von Polyoctamethylenpolyamin aus Korksäuredinitril (batch-Fahrweise)

### Beispiel 2

Der Versuch wurde analog Beispiel 1b durchgeführt. Als Ausgangsmaterial diente Korksäuredinitril. Während der vier Stunden Reaktionszeit wurden kontinuierlich pro Stunde 1,8 Mole Wasserstoff pro Mol Adiponitril durch das Druckgefäß geleitet und entsorgt. Flüssiges Kondensat wurde in das Druckgefäß zurückgeführt.

Die Reaktionsausträge wurden gaschromatographisch (Gewichts-%) und durch Gelpermeationschromatographie (Absoluteichung durch Messung von definierten Polyamin-Standards) analysiert. Die Analysenergebnisse sind in Tabelle 3 zusammengefasst

**Tabelle 3**

| T [°C] | 1,8-Diaminooctan | Korksäuredinitril | Dimer | Sonstige | Hochsieder | Mw | PDI |
|---|---|---|---|---|---|---|---|
| 160 | 17 | 0 | 20 | 3 | 60 | 570 | 3,2 |

### 6.4. Einstufige Herstellung von Polyhexamethylenpolyamin aus 6-Aminocapronitril (6-ACN) (kontinuierliche Fahrweise)

### Beispiel 3

Der Versuch wurde analog Beispiel 1c durchgeführt. 6-Aminocapronitril wurde kontinuierlich von unten nach oben über einen Katalysator geleitet, der in einem Rohrreaktor enthalten war. Als Katalysator wurden 378g aktivierter Kobaltkatalysator mit einem Strangdurchmesser von 4 mm eingesetzt, dessen Herstellung in der EP-A-0636409 als Katalysator A auf Seite 3, Zeile 35-44 beschrieben ist. Der Druck betrug 50 bar, die Temperatur 170°C. Die Katalysatorbelastung lag bei 0,1 kg/Lh 6-Aminocapronitril. Es wurden 2 Mole Wasserstoff pro Mol 6-Aminocapronitril durch den Reaktor geleitet (Abgasfahrweise). Der Rohaustrag war ein weißer, wachsartiger Feststoff. Die Zusammensetzung des Rohaustrags ist in Tabelle 4 zusammengefasst. Die Molgewichtsbestimmung erfolgte nach destillativer Abtrennung von HMI, HMD, Aminohexyl-HMI und Bis-HMD.

**Tabelle 4**

| T [°C] | HMI | HMD | Aminohexyl-HMI | Bis-HMD | HMI-HMD-HMI | HMI-(HMD)2 | Sonstige | Hochsieder | M_{w} | PDI |
|---|---|---|---|---|---|---|---|---|---|---|
| 170 | 3 | 1 | 4 | 1 | 5 | 3 | 8 | 75 | 1634 | 1,8 |

Die Analyse des Reaktionsaustrages zeigte, dass bei vollständigem 6-ACN-Umsatz und praktisch vollständigem HMD-Umsatz Polyhexamethylenpolyamin-Molgewichte von rund 1634 g/mol erreicht wurden.

### 6.5. Einstufige Herstellung von Polyamin aus zweifach cyanethyliertem Isophorondiamin (IPDA) (konti-Fahrweise)

### Beispiel 4

Der Versuch wurde analog zur einstufigen Herstellung von Polyhexamethylenpolyamin in kontinuierlicher Fahrweise durchgeführt (analog Versuch 1c). Dazu wurde zweifach cyanethyliertes IPDA kontinuierlich von unten nach oben über einen Katalysator geleitet. Als Katalysator wurden 378g aktivierter Kobaltkatalysator mit einem Strangdurchmesser von 4 mm eingesetzt, dessen Herstellung in der EP-A-0636409 als Katalysator A auf seite 3, Zeile 35-44 beschrieben ist. Der Druck betrug 50 bar, die Temperatur 160°C. Die Reaktion wurde in Gegenwart von THF als Lösungsmittel durchgeführt. Das Edukt wurde in 50 Gewichts-% THF gelöst. Die Katalysatorbelastung lag bei 0,1 kg/Lh Edukt, das in THF gelöst ist. Es wurden 12 Mole Wasserstoff pro Mol cyanethyliertem IPDA durch den Reaktor geleitet (Abgasfahrweise). Der Rohaustrag war zähflüssig und klar. Die Zusammensetzung des Rohaustrags ist in Tabelle 5 zusammengefasst. Die Molgewichtsbestimmung erfolgte nach destillativer Abtrennung von THF, 3,3,5-Trimethyl-7-azabicyclo[3.2.1]octan (IPDA-Ring) und IPDA.
zweifach cyanethyliertes IPDA

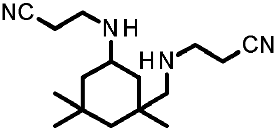

**Tabelle 5**

| T [°C] | THF | IPDA-Ring | IPDA | Sonstige | Hochsieder | Mw | PDI |
|---|---|---|---|---|---|---|---|
| 160 | 15 | 2 | 1 | 6 | 76 | 1096 | 1,8 |

### 6.6. Einstufige Herstellung von Polyaminen aus cyanethylierten Vorläufermolekülen (batch-Fahrweise)

Die Beispiele 5 bis 10 wurden analog Beispiel 1b durchgeführt.

### Beispiel 5

Als Einsatzstoff wurden 70 g N,N'-Bis-(2-cyanethyl)-piperazin gelöst in 70 g THF unter Stickstoff im Druckgefäß vorgelegt. 50 g des aktivierten Katalysators wurden in einem "Metallkäfig" fixiert, der von dem gerührten Reaktionsgemisch durchströmt wurde. Die Polymerisation erfolgte 5 Stunden lang bei 160 °C und 50 bar Gesamtdruck. Die Reaktionsausträge wurden gaschromatographisch (Gewichts-%) und durch Gelpermeationschromatographie (Absoluteichung durch Messung von definierten Polyamin-Standards) analysiert. Die Zusammensetzung des Rohaustrags ist in Tabelle 6 zusammengefasst.
N,N'-Bis-(2-cyanethyl)-piperazin

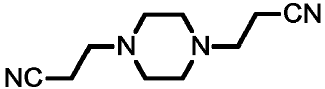

**Tabelle 6**

| T [°C] | THF | N-(3-Aminopropyl)-piperazin | N,N'-Bis-(3-aminopropyl)-piperazin | Sonstige | Hochsieder | Mw | PDI |
|---|---|---|---|---|---|---|---|
| 160 | 35 | 1 | 7 | 5 | 52 | 930 | 1,8 |

### Beispiel 6

Als Einsatzstoff wurden 60 g N-(2-Cyanethyl)-piperazin gelöst in 60 g THF unter Stickstoff im Druckgefäß vorgelegt. 50 g des aktivierten Katalysators wurden in einem "Metallkäfig" fixiert, der von dem gerührten Reaktionsgemisch durchströmt wurde. Die Polymerisation erfolgte 5 Stunden lang bei 160 °C und 50 bar Gesamtdruck. Die Reaktionsausträge wurden gaschromatographisch (Gewichts-%) und durch Gelpermeationschromatographie (Absoluteichung durch Messung von definierten Polyamin-Standards) analysiert. Die Zusammensetzung des Rohaustrags ist in Tabelle 7 zusammengefasst.
N-(2-Cyanethyl)-piperazin

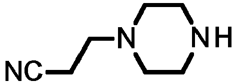

**Tabelle 7**

| T [°C] | THF | N-(3-Aminopropyl)-piperazin | Dimer | Sonstige | Hochsieder | Mw | PDI |
|---|---|---|---|---|---|---|---|
| 160 | 32 | 10 | 19 | 7 | 32 | 400 | 1,6 |

### Beispiel 7

Als Einsatzstoff wurden 70 g N,N'-Bis-(2-cyanethyl)-1,3-phenylendiamin gelöst in 70 g THF unter Stickstoff im Druckgefäß vorgelegt. 50 g des aktivierten Katalysators wurden in einem "Metallkäfig" fixiert, der von dem gerührten Reaktionsgemisch durchströmt wurde. Die Polymerisation erfolgte 5 Stunden lang bei 160°C und 50 bar Gesamtdruck. Die Reaktionsausträge wurden gaschromatographisch (Gewichts-%) und durch Gelpermeationschromatographie (Absoluteichung durch Messung von definierten Polyamin-Standards) analysiert. Die Zusammensetzung des Rohaustrags ist in Tabelle 8 zusam mengefasst.
N,N'-Bis-(2-cyanethyl)-1,3-phenylendiamin

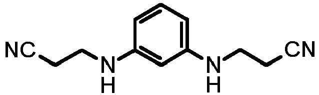

**Tabelle 8**

| T [°C] | THF | N-(3-aminpropyl)-1,3-phenylendiamin | N,N'-Bis-(3-aminpropyl)-1,3-phenylendiamin | Sonstige | Hochsieder | Mw | PDI |
|---|---|---|---|---|---|---|---|
| 160 | 31 | 12 | 8 | 9 | 40 | 440 | 1,8 |

### Beispiel 8

Als Einsatzstoff wurden 70 g N,N'-Bis-(2-cyanethyl)-2,4-toluylendiamin gelöst in 70 g THF unter Stickstoff im Druckgefäß vorgelegt. 50 g des aktivierten Katalysators wurden in einem "Metallkäfig" fixiert, der von dem gerührten Reaktionsgemisch durchströmt wurde. Die Polymerisation erfolgte 5 Stunden lang bei 160°C und 50 bar Gesamtdruck. Die Reaktionsausträge wurden gaschromatographisch (Gewichts-%) und durch Gelpermeationschromatographie (Absoluteichung durch Messung von definierten Polyamin-Standards) analysiert. Die Zusammensetzung des Rohaustrags ist in Tabelle 9 zusammengefasst.
N,N'-Bis-(2-cyanethyl)-2,4-toluylendiamin

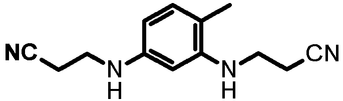

**Tabelle 9**

| T [°C] | THF | N,N'-Bis-(3-aminpropyl)-2,4-toluylendiamin | Sonstige | Hochsieder | Mw | PDI |
|---|---|---|---|---|---|---|
| 160 | 30 | 28 | 22 | 20 | 350 | 1,7 |

### Beispiel 9

Als Einsatzstoff wurden 70 g N,N'-Bis-(2-cyanethyl)-anilin gelöst in 70 g THF unter Stickstoff im Druckgefäß vorgelegt. 50 g des aktivierten Katalysators wurden in einem "Metallkäfig" fixiert, der von dem gerührten Reaktionsgemisch durchströmt wurde. Die Polymerisation erfolgte 5 Stunden lang bei 160 °C und 50 bar Gesamtdruck. Die Reaktionsausträge wurden gaschromatographisch (Gewichts-%) und durch Gelpermeationschromatographie (Absoluteichung durch Messung von definierten Polyamin-Standards) analysiert. Die Zusammensetzung des Rohaustrags ist in Tabelle 10 zusammengefasst.
N,N'-Bis-(2-cyanethyl)-anilin

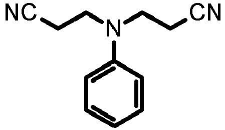

**Tabelle 10**

| T [°C] | THF | N,N'-Bis-(3-aminpropyl)-anilin | Sonstige | Hochsieder | Mw | PDI |
|---|---|---|---|---|---|---|
| 160 | 35 | 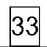 | 27 | 5 | 1097 | 2,6 |

### Beispiel 10

Als Einsatzstoff wurden 70 g N,N'-Bis-(2-cyanethyl)-methyldiamincyclohexan gelöst in 70 g THF unter Stickstoff im Druckgefäß vorgelegt. 50 g des aktivierten Katalysators wurden in einem "Metallkäfig" fixiert, der von dem gerührten Reaktionsgemisch durchströmt wurde. Die Polymerisation erfolgte 5 Stunden lang bei 160 °C und 50 bar Gesamtdruck. Die Reaktionsausträge wurden gaschromatographisch (Gewichts-%) und durch Gelpermeationschromatographie (Absoluteichung durch Messung von definierten Polyamin-Standards) analysiert. Die Zusammensetzung des Rohaustrags ist in Tabelle 11 zusammengefasst.
N,N'-Bis-(2-cyanethyl)-methyldiamincyclohexan

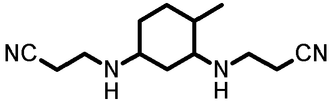

**Tabelle 11**

| T [°C] | THF | N,N'-Bis-(3-aminpropyl)-methyldiaminocyclohexan | Sonstige | Hochsieder | Mw | PDI |
|---|---|---|---|---|---|---|
| 160 | 31 | 33 | 16 | 53 | 593 | 1,7 |

## Patentansprüche

1. Verfahren zur Herstellung von Polyaminen, **dadurch gekennzeichnet, dass** man Verbindungen der Formel (I)
NC-Zₘ-(X)ₙ-Y (I)
wobei
Z der Gruppe CH₂-CH₂-NR- entspricht,
m = 0 oder 1 ist,
wobei im Fall von m=0 NC- direkt an X gebunden ist.
im Fall von m=1 und n=1 NR an X gebunden ist und R= H oder CH₂-CH₂-CN ist, oder
im Falle von m=1 und n=0 R von NR entweder ausgewählt ist aus der Gruppe von CH₃, Phenyl, CH₂-CH₂-CN und CH₂-CH₂-CH₂-NH₂ oder der Stickstoff des NR Teil eines Heterocyclus ausgewählt aus der Gruppe von Piperazinen, (Benz)imidazolen, Pyrazolen, Triazolen und Diazepanen ist, bei dem ein anderer Stickstoff des Heterocycluses an Y gebunden ist,
X ausgewählt ist aus der Gruppe von mit Alkylgruppen substituierten oder unsubstituierten Methylen-, Ethylen-, Propylen-, Butylen-, Pentylen-, Hexylen-, Heptylen-, Octylen-, Nonanylen-, Decanylen-, Undecanylen-, Dodecanylengruppe, von mit Alkylgruppen substituierten oder unsubstituierten 1,2-, 1,3- oder 1,4- Benzylgruppen, 1,4'-Bicarbonylgruppen, 9,10-Anthracenylgruppen, mit Alkylgruppen substituierten oder unsubstituierten 1,2-, 1,3- oder 1,4-Cycloalkylgruppen, substituierter oder unsubstituierter Phenyl-N-Gruppe, von Piperazinen, (Benz)imidazolen, Pyrazolen, Triazolen und Diazepanen, wobei jeweils über die zwei Stickstoffatome des Rings die Bindung an CN bzw. Z und Y bzw. H erfolgt,einer bis zwei Methylengruppen, die durch lineares oder verzweigtes C₁- bis C₁₂-Alkyl, C₆- bis C₁₀-Aryl, C₇-bis C₁₂-Aralkyl, C₃- bis C₈-Cycloalkyl substituiert sein können und mindestens eine Methylengruppe durch O, NH oder NR ersetzt sein kann, wobei R die gleich Bedeutung wie oben hat und die C₁- bis C₁₂-Alkyl, C₆- bis C₁₀-Aryl, C₇- bis C₁₂-Aralkyl, C₃- bis C₈-Cycloalkylgruppen ihrerseits noch mit Alkylgruppen substituiert sein können,
n 0 oder eine natürliche Zahl von 1 bis 10 ist,
Y, für den Fall, dass m= 0 und n=1 oder höher ist, CN, CH₂-NH₂ oder CH₂-CH₂-CN ist oder
für den Fall, dass m=1 und n=1 oder höher ist, NH₂ oder NR-CH₂-CH₂-CN ist oder
für den Fall, dass m=1 und n=0 ist, H , CH₂-CH₂-CN, CH₂-CH₂-CH₂-NH₂ , mit Alkylgruppen substituierte oder unsubstituierte Benzyl oder Cycloalkylgruppe, ist oder
für den Fall, dass m=0 und n=0 ist, CH₂-CN, CH₂-CH₂-CN, CH₂-CH₂-CH₂-CN, CH₂-CH₂-CH₂-CH₂-CN, CH₂-CH₂-CH₂-CH₂-CH₂-CN, CH₂-NH₂, CH₂-CH₂-NH₂ CH₂-CH₂-CH₂-NH₂ ist,
einstufig bei Temperaturen im Bereich von 100 bis 200°C und Drücken im Bereich von 20 bis 200 bar in Gegenwart von Wasserstoff und einem, falls mehrere Reaktoren verwendet werden, dem gleichen, heterogenen Katalysator, der ein oder mehrere Elemente der 8. Nebengruppe des Periodensystems enthält, umsetzt.

2. Verfahren nach Anspruch 1, wobei das gesamte Verfahren in einem einzigen Reaktor durchgeführt wird.

3. Verfahren nach einem der Ansprüche 1 bis 2, wobei jede Reaktionsstufe des Verfahrens beim gleichen Druck und der gleichen Temperatur durchgeführt wird.

4. Verfahren nach einem der Ansprüche 1 bis 3, wobei die Verbindungen der Formel I Dinitrile der Formel la
NC-(-X-)ₙ-CN (Ia)
oder
NC-CH₂-CH₂-NR-(-X-)ₙ-NH-CH₂-CH₂-CN (IIa)
sind, wobei R, n, X, die in Anspruch 1 genannte Bedeutung haben.

5. Verfahren nach einem der Ansprüche 1 bis 3, wobei die Verbindung der Formel I Aminonitrile der Formel Ib
NC-(-X)ₙ-CH₂NH₂, (Ib)
NC-(-X)ₙ (Ib')
oder
NC-CH₂-CH₂-NR-(-X)ₙ-NH₃ (IIb)
sind, wobei R, n und X, die in Anspruch 1 genannte Bedeutung haben.

6. Verfahren nach einem der Ansprüche 1 bis 5, wobei die Elemente des eingesetzten heterogenen Katalysators ausgewählt sind aus der Gruppe von Co, Ni und/oder Cu.

7. Verfahren nach einem der Ansprüche 1 bis 6, wobei der eingesetzt, reduzierte, heterogene Katalysator ein Co-Vollkontaktkatalysator mit bis zu 99 Gew.-% Co ist.

8. Verfahren nach einem der Ansprüche 1 bis 4 und 6 bis 7, wobei die eingesetzten Dinitrile ausgewählt sind aus der Gruppe von Malononitril, Succinonitril, Glutaronitril, Adiponitril, Suberonitril, 2-Methylmalononitril, 2-Methylsuccinonitril, 2-Methylglutaronitril, 1,2-, 1,3- und 1,4-Dicyanobenzol, 9,10-Anthracenodicarbonitril und 4,4'-Biphenyldicarbonitril.

9. Verfahren nach einem der Ansprüche 1 bis 3 und 5 bis 7, wobei die eingesetzten Aminonitrile ausgewählt sind aus der Gruppe von 6-Aminocapronitril, 4'-Aminobutyronitril, 3'-Aminopropionitril und 2'-Aminoacetonitril.

10. Verfahren nach einem der Ansprüche 1 bis 9, wobei man einen Überschuss an Wasserstoff, bezogen auf die theoretisch zur Hydrierung der Nitrilgruppen notwendige Wasserstoff-Menge, von 1 bis 5 Molen in die Flüssig- oder Gasphase des ersten Reaktors (R1) einleitet und überschüssigen Wasserstoff zusammen mit Ammoniak, der bei der Umsetzung entsteht, aus dem letzten Reaktor ableitet.

11. Verfahren nach einem der Ansprüche 1 bis 10, wobei der Ammoniak aus dem ausgeleiteten Ammoniak/Wasserstoffgemisch abgetrennt, aus dem Verfahren ausgeschleust und der abgetrennte Wasserstoff ganz oder teilweise in den Reaktor zurückgeführt wird.

12. Verfahren nach einem der Ansprüchen 1 bis 11, wobei die Trennung des Ammoniak/Wasserstoff-Gasgemisches durch Destillation oder nach Kühlung durch Phasentrennung erfolgt.

13. Verfahren nach einem der Ansprüche 1 bis 12, wobei ein Teil des abgetrennten Ammoniaks in den ersten Reaktor (R1) zurückgeführt wird.

14. Verfahren nach einem der Ansprüche 1 bis 13, wobei das Verfahren diskontinuierlich oder kontinuierlich durchgeführt wird.

15. Verfahren nach einem der Ansprüche 1 bis14, wobei es sich bei dem oder den Reaktoren um Rohrreaktoren oder Rohrbündelreaktoren handelt.

16. Verfahren nach einem der Ansprüche 1 bis 15, wobei die aus dem Polyamin-Rohaustrag nicht umgesetzten Nitrile, Aminonitrile, und den daraus entstehenden Diaminen und Polyamin-Oligomere destillativ abgetrennt und in den ersten Reaktor (R1) zurückgeführt werden.

## Claims

1. A process for preparing polyamines, which comprises converting compounds of the formula (I)
NC-Zₘ-(X)ₙ-Y (I)
where
Z corresponds to the CH₂-CH₂-NR- group,
m = 0 or 1,
where in the case that m = 0 NC- is bonded directly to X,
in the case that m = 1 and n = 1 NR is bonded to X and R = H or CH₂-CH₂-CN, or
in the case that m = 1 and n = 0 either R of NR is selected from the group consisting of CH₃, phenyl, CH₂-CH₂-CN and CH₂-CH₂-CH₂-NH₂ or the nitrogen of the NR is part of a heterocycle selected from the group of piperazines, (benz)imidazoles, pyrazoles, triazoles and diazepanes, in which another nitrogen of the heterocycle is bonded to Y,
X is selected from the group of alkyl group-substituted or unsubstituted methylene, ethylene, propylene, butylene, pentylene, hexylene, heptylene, octylene, nonanylene, decanylene, undecanylene, dodecanylene groups, of alkyl group-substituted or unsubstituted 1,2-, 1,3- or 1,4-benzyl groups, 1,4'-bicarbonyl groups, 9,10-anthracenyl groups, alkyl group-substituted or unsubstituted 1,2-, 1,3- or 1,4-cycloalkyl groups, substituted or unsubstituted phenyl-N group, of piperazines, (benz)imidazoles, pyrazoles, triazoles and diazepanes, where the bond to CN or Z and Y or H in each case is via the two nitrogen atoms of the ring, one to two methylene groups that may be substituted by linear or branched C₁- to C₁₂-alkyl, C₆- to C₁₀-aryl, C₇- to C₁₂-aralkyl, C₃- to C₈-cycloalkyl, and at least one methylene group may be replaced by 0, NH or NR, where R has the same definition as above and the C₁- to C₁₂-alkyl, C₆- to C₁₀-aryl, C₇- to C₁₂-aralkyl, C₃- to C₈-cycloalkyl groups may in turn be substituted by alkyl groups,
n is 0 or a natural number from 1 to 10,
Y in the case that m = 0 and n = 1 or higher is CN, CH₂-NH₂ or CH₂-CH₂-CN or
in the case that m = 1 and n = 1 or higher is NH₂ or NR-CH₂-CH₂-CN or
in the case that m = 1 and n = 0 is H, CH₂-CH₂-CN, CH₂-CH₂-CH₂-NH₂, alkyl group-substituted or unsubstituted benzyl or cycloalkyl group, or
in the case that m = 0 and n = 0 is CH₂-CN, CH₂-CH₂-CN, CH₂-CH₂-CH₂-CN, CH₂-CH₂-CH₂-CH₂-CN, CH₂-CH₂-CH₂-CH₂-CH₂-CN, CH₂-NH₂, CH₂-CH₂-NH₂, CH₂-CH₂-CH₂-NH₂,
in one stage at temperatures in the range from 100 to 200°C and pressures in the range from 20 to 200 bar in the presence of hydrogen and a heterogeneous catalyst, the same heterogeneous catalyst if two or more reactors are used, comprising one or more elements of transition group 8 of the Periodic Table.

2. The process according to claim 1, wherein the entire process is conducted in a single reactor.

3. The process according to either of claims 1 and 2, wherein each reaction stage of the process is conducted at the same pressure and the same temperature.

4. The process according to any of claims 1 to 3, wherein the compounds of the formula I are dinitriles of the formula Ia
NC-(-X-)ₙ-CN (Ia)
or
NC-CH₂-CH₂-NR- (-X-)ₙ-NH-CH₂-CH₂-CN (IIa)
where R, n, X are as defined in claim 1.

5. The process according to any of claims 1 to 3, wherein the compounds of the formula I are amino nitriles of the formula Ib
NC-(-X)ₙ-CH₂NH₂, (Ib)
NC-(-X)ₙ (Ib')
or
NC-CH₂-CH₂-NR-(-X)ₙ-NH₃ (IIb)
where R, n and X are as defined in claim 1.

6. The process according to any of claims 1 to 5, wherein the elements in the heterogeneous catalyst used are selected from the group of Co, Ni and/or Cu.

7. The process according to any of claims 1 to 6, wherein the reduced heterogeneous catalyst used is an unsupported Co catalyst with up to 99% by weight of Co.

8. The process according to any of claims 1 to 4 and 6 to 7, wherein the dinitriles used are selected from the group of malononitrile, succinonitrile, glutaronitrile, adiponitrile, suberonitrile, 2-methylmalononitrile, 2-methylsuccinonitrile, 2-methylglutaronitrile, 1,2-, 1,3- and 1,4-dicyanobenzene, 9,10-anthracenodicarbonitrile and 4,4'-biphenyldicarbonitrile.

9. The process according to any of claims 1 to 3 and 5 to 7, wherein the amino nitriles used are selected from the group of 6-aminocapronitrile, 4'-aminobutyronitrile, 3'-aminopropionitrile and 2'-aminoacetonitrile.

10. The process according to any of claims 1 to 9, wherein an excess of hydrogen, based on the amount of hydrogen theoretically needed for hydrogenation of the nitrile groups, of 1 to 5 moles is introduced into the liquid or gas phase of the first reactor (R1), and excess hydrogen together with ammonia formed in the reaction is removed from the last reactor.

11. The process according to any of claims 1 to 10, wherein the ammonia is separated from the ammonia/hydrogen mixture discharged and discharged from the process, and the hydrogen separated off is wholly or partly recycled into the reactor.

12. The process according to any of claims 1 to 11, wherein the ammonia/hydrogen gas mixture is separated by distillation or after cooling by phase separation.

13. The process according to any of claims 1 to 12, wherein a portion of the ammonia separated off is recycled into the first reactor (R1).

14. The process according to any of claims 1 to 13, wherein the process is conducted batchwise or continuously.

15. The process according to any of claims 1 to 14, wherein the reactor is/reactors are tubular reactors or shell and tube reactors.

16. The process according to any of claims 1 to 15, wherein the unconverted nitriles and amino nitriles from the crude polyamine output and the diamines and polyamine oligomers formed therefrom are separated off by distillation and recycled into the first reactor (R1).

## Revendications

1. Procédé de fabrication de polyamines, **caractérisé en ce que** des composés de la formule (I) :
NC-Zₘ-(X)ₙ-Y (I)
dans laquelle
Z correspond au groupe CH₂-CH₂-NR-,
m = 0 ou 1,
dans le cas où m = 0, NC- étant directement relié à X,
dans le cas où m = 1 et n = 1, NR étant relié à X et R = H ou CH₂-CH₂-CN, ou
dans le cas où m = 1 et n = 0, le R de NR étant choisi dans le groupe constitué par CH₃, phényle, CH₂-CH₂-CN et CH₂-CH₂-CH₂-NH₂, ou l'azote du NR faisant partie d'un hétérocycle choisi dans le groupe constitué par les pipérazines, les (benz)imidazoles, les pyrazoles, les triazoles et les diazépanes, dans lequel un autre azote de l'hétérocycle est relié à Y,
X est choisi dans le groupe constitué par les groupes méthylène, éthylène, propylène, butylène, pentylène, hexylène, heptylène, octylène, nonanylène, décanylène, undécanylène, dodécanylène substitués avec des groupes alkyle ou non substitués, les groupes 1,2-, 1,3- ou 1,4-benzyle substitués avec des groupes alkyle ou non substitués, les groupes 1,4'-bicarbonyle, les groupes 9,10-anthracényle, les groupes 1,2-, 1,3- ou 1,4-cycloalkyle substitués avec des groupes alkyle ou non substitués, le groupe phényl-N- substitué ou non substitué, les pipérazines, les (benz)imidazoles, les pyrazoles, les triazoles et les diazépanes, la liaison à CN ou Z et Y ou H ayant lieu à chaque fois par l'intermédiaire des deux atomes d'azote du cycle, un à deux groupes méthylène, qui peuvent être substitués par alkyle en C₁ à C₁₂ linéaire ou ramifié, aryle en C₆ à C₁₀, aralkyle en C₇ à C₁₂, cycloalkyle en C₃ à C₈, et au moins un groupe méthylène peut être remplacé par 0, NH ou NR, R ayant la même signification que précédemment, et les groupes alkyle en C₁ à C₁₂, aryle en C₆ à C₁₀, aralkyle en C₇ à C₁₂, cycloalkyle en C₃ à C₈ pouvant eux-mêmes encore être substitués avec des groupes alkyle,
n vaut 0 ou est un nombre naturel de 1 à 10,
Y dans le cas où m = 0 et n = 1 ou plus, représente CN, CH₂-NH₂ ou CH₂-CH₂-CN, ou
dans le cas où m = 1 et n = 1 ou plus, représente NH₂ ou NR-CH₂-CH₂-CN, ou
dans le cas où m = 1 et n = 0, représente H, CH₂-CH₂-CN, CH₂-CH₂-CH₂-NH₂, un groupe benzyle ou cycloalkyle substitué avec des groupes alkyle ou non substitué, ou
dans le cas où m = 0 et n = 0, représente CH₂-CN, CH₂-CH₂-CN, CH₂-CH₂-CH₂-CN, CH₂-CH₂-CH₂-CH₂-CN, CH₂-CH₂-CH₂-CH₂-CH₂-CN, CH₂-NH₂, CH₂-CH₂-NH₂, CH₂-CH₂-CH₂-NH₂,
sont mis en réaction en une étape à des températures dans la plage allant de 100 à 200 °C et des pressions dans la plage allant de 20 à 200 bar en présence d'hydrogène et d'un, si plusieurs réacteurs sont utilisés, du même, catalyseur hétérogène, qui contient un ou plusieurs éléments du groupe de transition 8 du tableau périodique.

2. Procédé selon la revendication 1, dans lequel l'ensemble du procédé est réalisé dans un réacteur unique.

3. Procédé selon l'une quelconque des revendications 1 à 2, dans lequel chaque étape de réaction du procédé est réalisée à la même pression et à la même température.

4. Procédé selon l'une quelconque des revendications 1 à 3, dans lequel les composés de la formule I sont des dinitriles de la formule la
NC-(-X-)ₙ-CN (Ia)
ou
NC-CH₂-CH₂-NR-(-X-)ₙ-NH-CH₂-CH₂-CN (IIa)
dans lesquelles R, n, X ont la signification indiquée dans la revendication 1.

5. Procédé selon l'une quelconque des revendications 1 à 3, dans lequel les composés de la formule I sont des aminonitriles de la formule Ib :
NC-(-X-)ₙ-CH₂NH₂ (Ib)
NC-(-X)ₙ (Ib')
ou
NC-CH₂-CH₂-NR-(-X)ₙ-NH₃ (IIb)
dans lesquelles R, n et X ont la signification indiquée dans la revendication 1.

6. Procédé selon l'une quelconque des revendications 1 à 5, dans lequel les éléments du catalyseur hétérogène utilisé sont choisis dans le groupe constitué par Co, Ni et/ou Cu.

7. Procédé selon l'une quelconque des revendications 1 à 6, dans lequel le catalyseur hétérogène réduit utilisé est un co-catalyseur de contact entier contenant jusqu'à 99 % en poids de Co.

8. Procédé selon l'une quelconque des revendications 1 à 4 et 6 à 7, dans lequel les dinitriles utilisés sont choisis dans le groupe constitué par le malononitrile, le succinonitrile, le glutaronitrile, l'adiponitrile, le subéronitrile, le 2-méthylmalononitrile, le 2-méthylsuccinonitrile, le 2-méthylglutaronitrile, le 1,2-, 1,3- et 1,4-dicyanobenzène, le 9,10-anthracénodicarbonitrile et le 4,4'-biphényldicarbonitrile.

9. Procédé selon l'une quelconque des revendications 1 à 3 et 5 à 7, dans lequel les aminonitriles utilisés sont choisis dans le groupe constitué par le 6-aminocapronitrile, le 4'-aminobutyronitrile, le 3'-aminopropionitrile et le 2'-aminoacétonitrile.

10. Procédé selon l'une quelconque des revendications 1 à 9, dans lequel un excès d'hydrogène, par rapport à la quantité d'hydrogène nécessaire théoriquement pour l'hydrogénation des groupes nitrile, de 1 à 5 moles est introduit dans la phase liquide ou gazeuse du premier réacteur (R1), et l'hydrogène en excès est déchargé du dernier réacteur conjointement avec de l'ammoniac, qui se forme lors de la réaction.

11. Procédé selon l'une quelconque des revendications 1 à 10, dans lequel l'ammoniac est séparé du mélange ammoniac/hydrogène déchargé, évacué du procédé et l'hydrogène séparé est recyclé en totalité ou en partie dans le réacteur.

12. Procédé selon l'une quelconque des revendications 1 à 11, dans lequel la séparation du mélange gazeux ammoniac/hydrogène a lieu par distillation ou après refroidissement par séparation de phases.

13. Procédé selon l'une quelconque des revendications 1 à 12, dans lequel une partie de l'ammoniac séparé est recyclé dans le premier réacteur (R1).

14. Procédé selon l'une quelconque des revendications 1 à 13, dans lequel le procédé est réalisé de manière discontinue ou continue.

15. Procédé selon l'une quelconque des revendications 1 à 14, dans lequel le ou les réacteurs consistent en des réacteurs tubulaires ou des réacteurs à faisceau de tubes.

16. Procédé selon l'une quelconque des revendications 1 à 15, dans lequel les nitriles non réagis, les aminonitriles, issus de la sortie brute de polyamine, et les diamines et oligomères de polyamines formés à partir de ceux-ci sont séparés par distillation et recyclés dans le premier réacteur (R1).
